# FASCICULE DE BREVET EUROPEEN

(11) **EP 2 825 524 B1**
(45) Date de publication et mention de la délivrance du brevet: **26.09.2018**
(21) Numéro de dépôt: 13715332.6
(22) Date de dépôt: 15.03.2013
(51) Int. Cl.: C07C 229/76, C07C 323/52, C07C 319/20, C07C 319/28, C07C 227/18, C08B 37/00, B01J 2/04, C07C 323/58, C08L 5/00, A23K 20/22, A23K 20/24, A23K 50/75

(54) **COMPOSITIONS PULVERULENTES D'UN COMPLEXE ENTRE UN ACIDE ET UN METAL ET LEUR PROCEDE DE PREPARATION**
PULVERZUSAMMENSETZUNGEN AUS EINEM KOMPLEX ZWISCHEN EINER SÄURE UND EINEM METALL SOWIE VERFAHREN ZUR DEREN HERSTELLUNG
POWDER COMPOSITIONS OF A COMPLEX BETWEEN AN ACID AND A METAL AND METHOD FOR PREPARING SAME

(30) Priorité: 16.03.2012 FR 1252423
(43) Date de publication de la demande: 21.01.2015
(60) Demande divisionnaire: 17164032.9
(73) Titulaire: Innov'ia 3i, 63380 Pontaumur (FR)
(72) Inventeur: BUISSON, Pierre, F-17140 Lagord (FR); HUET, Robert, F-75015 Paris (FR); FOURNIER, Sébastien, F-17230 Andilly (FR); VENDEVILLE, Jean-Eudes, F-17180 Perigny (FR)
(74) Mandataire: Grosset-Fournier, Chantal Catherine
(86) Numéro de dépôt international: PCT/FR2013/050549
(87) Numéro de publication internationale: WO 2013/136030

(56) Documents cités:
- EP-A1- 0 100 974
- EP-A1- 0 140 865
- EP-A1- 1 566 373
- WO-A1-2004/012707
- DE-A1- 19 707 380
- US-A- 4 067 994
- US-A- 4 335 257
- US-A- 4 579 962
- US-A1- 2006 251 765
- US-B1- 6 287 627
- R. P. PATEL ET AL: "Spray drying technology: an overview", INDIAN JOURNAL OF SCIENCE AND TECHNOLOGY, vol. 2, no. 10, 1 octobre 2009 (2009-10-01), pages 44-47, XP055049216,

## Description

La présente invention a pour objet des compositions pulvérulentes d'un complexe entre un acide et un métal et leur procédé de préparation.

La méthionine, acide aminé essentiel, et l'HMTBA, un analogue de la méthionine, trouvent des applications largement étendues chez l'homme en tant que complément alimentaire ou médicament, ainsi qu'en nutrition animale. Leurs sels métalliques, par exemple calciques, magnésiques ou de zinc, sous forme solide, peuvent être préférés. Ceux-ci permettent aussi de combler des carences en éléments ou oligoéléments. Le sel d'HMTBA le plus connu est le sel dicalcique, comprenant deux moles d'équivalent d'HMTBA par mole de calcium répondant à la formule (HMTBA)₂Ca.

On connait selon EP140865A un procédé de préparation de sels de calcium d'HMTBA, consistant en plus de deux et moins de dix moles d'équivalent d'HMTBA par mole de calcium. Ces sels sont obtenus en mettant en réaction l'HMTBA avec une source de calcium choisie parmi l'oxyde calcium (CaO), l'hydroxyde de calcium (Ca(OH)₂), le carbonate de calcium (CaCO₃) ainsi qu'un sel d'HMTBA, par exemple le sel (HMTBA)₂ Ca. L'HMTBA est généralement en solution aqueuse fortement concentrée, à laquelle la source de calcium est mélangée, puis le milieu réactionnel ainsi obtenu est séché à une température de l'ordre de 70°C. Le milieu réactionnel d'HMTBA avec la source de calcium est cependant très visqueux et collant: il est donc très difficile à homogénéiser dans des mélangeurs ou réacteurs équipés de systèmes d'agitation classiques et, en fin de réaction, il est nécessaire de procéder à un séchage in-situ pour pouvoir vidanger le réacteur.

Un recyclage de sel de calcium d'HMTBA, par exemple le sel (HMTBA)₂Ca, à la source de calcium avant de la mettre en contact avec l'HMTBA permet d'améliorer la consistance du milieu réactionnel et facilite la mise en oeuvre du procédé comme cité dans EP140865A. Mais, comme enseigné par US 4 335 257, cette amélioration est observée pour une proportion pondérale d'au moins 20% dudit sel par rapport au milieu réactionnel, et pour parvenir à une consistance acceptable, il peut être nécessaire que cette proportion atteigne 80% du milieu réactionnel. Un tel taux de recyclage de produit fini dans le mélange réactionnel réduit considérablement la productivité d'une installation industrielle et contraint à un fort surdimensionnement du mélangeur / réacteur pour une capacité de production souhaitée.

WO03/011822A2 propose un procédé de préparation de sels d'acide organique, notamment de calcium, à partir d'un dit acide organique et d'hydroxyde de calcium et/ou d'oxyde de calcium, dans lequel on dépose l'acide organique sur un support inerte avant de lui ajouter la source de calcium. Malgré la présence de ce support, il est indispensable d'introduire les deux réactifs par ajouts successifs pour permettre un séchage du milieu réactionnel entre deux ajouts. Ce mode opératoire rallonge notablement le temps de séjour dans le mélangeur et contraint également à un fort surdimensionnement dudit mélangeur pour une capacité de production donnée. En outre, le support inerte se retrouve dans le produit fini sec où il représente de 30 à 50% en poids de la masse totale, ce qui réduit d'autant le titre en matière active et génère ainsi des surcoûts à l'utilisation du produit (stockage, transport, dosage, ...).

Une solution permettant en partie de contourner les obstacles précités, sans pour autant faire appel à un support ou autre excipient, pourrait être d'utiliser un procédé simple de préparation d'un ou de sels d'HMTBA au moyen d'une extrudeuse. L'avantage d'un tel procédé serait d'effectuer un mélange rapide de l'acide et du métal en un temps court et avec des forces de cisaillement qui permettraient d'extruder le sel d'HMTBA formé sans difficulté. Cependant ce type de procédé conduirait à la formation de vermicelles en sortie de la filière d'extrusion non compatibles avec les tailles particulaires recherchées dans les applications finales du sel d'HMTBA, par exemple l'élaboration de produits d'alimentation animale. De plus il resterait à sécher le produit, ce qui conduit en définitive à ajouter deux opérations unitaires complémentaires de séchage et broyage du sel d'HMTBA pour être utilisable.

FR 1469803, US 2006/0251765 et US 6287627 décrivent des procédés de préparation de sels entre l'anion d'un acide et un cation métallique, ledit procédé comprenant une étape permettant l'obtention d'une solution aqueuse dudit sel, et une étape de séchage de ladite solution aqueuse par atomisation.

Ces procédés en deux étapes permettent d'obtenir des sels sous forme de poudres, dont la granulométrie permet l'utilisation directe desdites poudres, par exemple pour l'élaboration de produits d'alimentation animale. Cependant, ces derniers introduisent en plus de l'étape de réaction une étape supplémentaire de séchage. De plus, ils n'autorisent pas un extrait sec du mélange réactionnel élevé ; un extrait sec élevé conduirait au bouchage des installations utilisées.

Ainsi, un but de la présente invention consiste à fournir des complexes entre un acide et un métal, sans faire appel à un support ou autre excipient, en une seule étape.

Un autre but de l'invention consiste à fournir, en continu, des complexes entre un acide et un métal, sous la forme de poudres stables, aisément manipulables et adaptées à l'application à laquelle lesdits complexes sont destinés.

Un autre but de l'invention consiste à fournir un procédé autorisant un extrait sec du mélange réactionnel élevé, sans bouchage.

Un autre but de l'invention consiste à fournir un procédé de préparation de complexes entre un acide et un métal, en continu.

La présente description concerne notamment une particule, essentiellement sous la forme d'une sphère homogène ou d'une fraction de sphère homogène, constituée essentiellement d'un complexe, notamment un sel, entre un acide ou un anion correspondant et au moins un métal ou un cation métallique correspondant, ladite particule étant substantiellement dépourvue d'acide ou d'anion non complexé et de métal ou de cation métallique non complexé, ledit acide étant différent de l'acide formique, l'acide acétique, et l'acide propionique, et ledit anion étant différent du formate, de l'acétate et du propionate.

Par particule, on entend un petit élément monolithique de matière qui est d'un seul tenant et qui n'est donc pas constitué d'une juxtaposition d'éléments plus petits.

Par sphère homogène, on entend une sphère ou un sphéroïde dans laquelle (lequel) on ne retrouve pas ledit acide ou anion, ou ledit métal ou cation, à l'état pur.

En particulier, une sphère homogène est une sphère ne comprenant qu'un seul complexe entre un acide et un métal déterminés, notamment qu'un seul sel entre un anion et un cation déterminés.

Par sphéroïde, on entend un solide dont la forme approche celle de la sphère.

Par l'expression «ledit acide ou anion, ou ledit métal ou cation, à l'état pur », on entend que ledit acide ou anion, ou ledit métal ou cation, n'est pas intimement mélangé au dit complexe, mais forme au contraire au moins un espace où il est trouvé pur.

Par fraction de sphère homogène, on entend tout fragment issu d'une sphère homogène brisée.

Par métal selon l'invention, on entend tout élément du tableau périodique, capable de former un ou des cations, ou un complexe avec un acide, et appartenant aux groupes des métaux notamment des métaux alcalins, alcalino-terreux, de transition et des métalloïdes.

Par complexe selon l'invention, on entend un composé comprenant au moins un atome de métal et au moins une molécule d'un acide tel que défini ci-dessus, dans lequel au moins un atome de la molécule d'acide est lié à l'atome de métal, ou aux atomes de métal pour la préparation de complexes mixtes, par une liaison chimique ou une interaction chimique. A titre d'illustration, une ou de telles liaisons ou une ou de telles interactions chimiques sont choisies parmi les liaisons ioniques, les liaisons de coordination, des liaisons de Van der Waals... Lorsque la liaison ou l'interaction chimique est une liaison ionique, le complexe est alors un sel entre un acide et un métal, respectivement sous la forme d'un anion correspondant au dit acide au moins une fois déprotoné et d'un cation métallique, fait d'un atome dudit métal.

Par « acide ou anion correspondant », on entend l'acide ou un anion formé par au moins une déprotonation dudit acide.

Par « métal ou cation métallique correspondant », on entend le métal ou un cation fait d'un atome dudit métal.

La présente description divulgue également une particule, essentiellement sous la forme d'une sphère homogène ou d'une fraction de sphère homogène, constituée essentiellement d'un complexe, notamment un sel, entre un acide ou un anion correspondant et au moins un métal ou un cation métallique correspondant,
ledit acide ou anion correspondant étant choisi dans le groupe comprenant l'acide 2-hydroxy-4-méthyl-thio-butanoïque (HMTBA), la méthionine, l'acide aspartique, les acides alginiques, les acides pectiniques, et les anions correspondants, en particulier le 2-hydroxy-4-méthyl-thio-butanoate, le méthioniate, l'aspartate, les alginates et les pectinates,
ledit métal ou cation métallique étant divalent ou trivalent,
ladite particule présentant une fraction amorphe dont la masse représente au moins 50%, en particulier au moins 70%, plus particulièrement au moins 90%, de la masse totale de ladite particule,
ladite particule étant substantiellement dépourvue d'acide ou d'anion non complexé et de métal ou de cation métallique non complexé.

L'invention a notamment pour objet une particule, sous la forme d'une sphère homogène ou d'une fraction de sphère homogène, constituée essentiellement d'un complexe, notamment un sel, entre un acide ou un anion correspondant et au moins un métal ou un cation métallique correspondant, ledit acide ou anion correspondant étant l'acide 2-hydroxy-4-méthyl-thio-butanoïque (HMTBA) ou le 2-hydroxy-4-méthyl-thio-butanoate, ledit métal ou cation métallique étant divalent, choisi parmi le Ca ou le Ca²⁺, ladite particule présentant une fraction amorphe dont la masse représente au moins 50%, en particulier au moins 70%, plus particulièrement au moins 90%, de la masse totale de ladite particule, ladite particule étant substantiellement dépourvue d'acide ou d'anion non complexé et de métal ou de cation métallique non complexé, le rapport de la masse dudit acide ou anion non complexé et/ou dudit au moins un métal ou cation non complexé sur la masse totale de ladite particule étant inférieure à 20 %, et variant en particulier de 0 à 5 %, plus particulièrement de 0 à 1 %.

Un complexe selon l'invention peut être un composé comprenant au moins un atome de métal et au moins une molécule d'un acide, en particulier un composé comprenant un atome de métal et au moins une molécule d'un acide, dans lequel :
- un atome de la molécule d'acide est lié à au moins un atome de métal, par une liaison ou interaction chimique, par exemple une liaison ionique, et
- un autre atome de la molécule d'acide est lié au dit atome de métal, par le même type de liaison ou d'interaction chimique, par exemple une liaison ionique.

Un complexe selon l'invention peut également être un composé comprenant au moins un atome de métal et au moins une molécule d'un acide, en particulier un composé comprenant un atome de métal et au moins une molécule d'un acide, dans lequel :
- un atome de la molécule d'acide est lié à au moins un atome de métal, par une liaison ou interaction chimique, par exemple une liaison ionique, et
- un autre atome de la molécule d'acide est lié au dit atome de métal, par un autre type de liaison ou d'interaction chimique, par exemple une liaison de coordination.

A titre d'illustration, un complexe de l'invention peut être représenté par la formule I suivante :

(Acide)ₙXₚY_{q} (I)

où X et Y représentent indépendamment l'un de l'autre un métal,
n est un nombre entier variant de 1 à 10, et
p et q sont des nombres entiers variant de 0 à 10, la somme de p et q variant de 1 à 10.

Un complexe entre un acide ou un anion correspondant et au moins un métal ou un cation métallique correspondant selon l'invention désigne notamment un sel, entre un anion et un cation.

A titre d'illustration, un sel de l'invention peut être représenté par la formule (II) suivante :

(Acide^{k-})ₙ(X¹⁺)ₚ(Y^{m+})_{q} (II)

où :
X et Y représentent indépendamment l'un de l'autre un métal,
k est un nombre entier variant de 1 à 7,
l et m sont des nombres entiers variant de 1 à 7,
n est un nombre entier variant de 1 à 10, et
p et q sont des nombres entiers variant de 0 à 10,
k, 1, m, n, p et q étant tels que : kn = lp + mq.

Ainsi, « Acide^{k-} » représente un anion d'un acide, ledit anion portant k charges négatives.
« X¹⁺» représente un cation métallique portant 1 charges positives.
« Y^{m+}» représente un cation métallique portant m charges positives.

En particulier, un sel de l'invention peut être représenté par la formule (III) suivante :

(Acide^{k-})ₙ(X¹⁺)ₚ (III)

où :
X représente un métal,
k est un nombre entier variant de 1 à 7, k étant en particulier égal à 1,
l est un nombre entier variant de 1 à 7,
n est un nombre entier variant de 1 à 10, et
p est un nombre entier variant de 0 à 10, étant en particulier égal à 1,
k, 1, n et p étant tels que : kn = lp.

De manière encore plus particulière, un sel de l'invention peut être représenté par la formule (IV) suivante :

(Acide⁻)₁(X¹⁺) (IV)

où :
X représente un métal,
l est un nombre entier variant de 1 à 7.

Selon un mode de réalisation avantageux, la présente description concerne une particule constituée essentiellement d'un complexe, notamment un sel, ledit complexe, notamment ledit sel, étant susceptible de précipiter en phase aqueuse, ladite phase aqueuse résultant du mélange entre un premier milieu aqueux, en particulier une solution aqueuse, contenant ledit acide et un deuxième milieu aqueux, en particulier une solution ou une suspension aqueuse contenant ledit métal ou cation, à une température variant de la température ambiante à 100°C, la masse dudit complexe, notamment du sel, sur la masse totale de ladite phase aqueuse variant de 15 à 90 %, en particulier de 40 à 75 %.

Ainsi, le premier milieu aqueux contient ledit acide. Le deuxième milieu aqueux contient ledit métal ou cation.

Par « complexe susceptible de précipiter en phase aqueuse », on entend qu'il existe au moins une température, dans l'intervalle température ambiante à 100°C, et au moins un rapport massique complexe sur masse totale de ladite phase aqueuse, dans l'intervalle 15 à 90%, en particulier 40 à 75%, tels que le mélange du premier milieu aqueux et du deuxième milieu aqueux, pour former ladite phase aqueuse, provoque la précipitation dudit complexe dans ladite phase aqueuse.

On entend par « précipitation du complexe dans la phase aqueuse» la création d'une phase solide, comprenant tout ou partie dudit complexe, à partir de ladite phase aqueuse, ladite phase aqueuse pouvant éventuellement encore contenir, après précipitation, une partie du complexe, en solution.

Selon un mode de réalisation avantageux, la présente invention concerne une particule, essentiellement sous la forme d'une sphère homogène ou d'une fraction de sphère homogène, dans laquelle ladite sphère est pleine, creuse, ou contient des ouvertures.

Par sphère pleine, on entend une sphère formée de matière substantiellement dépourvue de cavités.

Par sphère creuse, on entend toute sphère formée de matière pourvue d'au moins une cavité ou vacuole.

Par « sphère contenant des ouvertures », on entend une sphère creuse dont au moins une des cavités ou vacuole communique avec l'extérieur de ladite sphère.

Des exemples de telles formes de particules peuvent être trouvés dans Masters, K. Spray drying Handbook ; Ed. Longman Scientific and Technical, 1988, p 323, figure 8.5 a à f.

Il est à noter que les particules illustrées dans ledit ouvrage n'ont pas la même composition que les particules de la présente invention.

Selon un mode de réalisation avantageux, la présente invention concerne une particule dans laquelle le rapport de la masse dudit acide ou anion non complexé et/ou dudit au moins un métal ou cation non complexé sur la masse totale de ladite particule est inférieure à 20 %, et varie en particulier de 0 à 5 %, plus particulièrement de 0 à 1 %.

Si la masse dudit acide ou anion non complexé est supérieur à 20%, la quantité dudit acide ou anion non complexée ne permet pas d'obtenir des particules sèches et ne conduit à l'obtention d'une composition pulvérulente. De plus, la quantité de sel formée n'est plus suffisante et ne permet pas aux particules obtenues de remplir les besoins recherchés dans les applications ultérieures desdites particules.

Selon un mode avantageux le rapport de la masse dudit acide ou anion non complexé sur la masse totale de la dite particule est nulle (0%) et la masse dudit métal ou cation sur la masse totale de la dite particule est nulle ou compris entre 0 et 5%, de préférence entre 0 et 1%, de façon à assurer une conversion totale dudit acide.

Selon un mode de réalisation avantageux, la présente invention concerne une particule dans laquelle, pour tout élément de 1 µm² considéré à la surface de ladite particule, le rapport de la masse dudit complexe, notamment dudit sel, sur la masse totale de la matière occupant ledit volume varie de 70% à 100 %.

Ledit rapport peut par exemple être mesuré par analyse atomique en microscopie électronique à balayage sur une surface de 1 µm².

Si ledit rapport est inférieur à 70%, cela traduit une inhomogénéité de ladite particule, c'est-à-dire un mélange non homogène entre le dit acide ou anion et le dit métal ou cation à l'échelle de la particule.

A contrario, si ledit rapport est supérieur à 70%, la particule est considérée comme homogène, c'est-à-dire que le mélange entre le dit acide ou anion et le dit métal ou cation est homogène à l'échelle de la particule.

Selon un mode de réalisation avantageux, la présente invention concerne une particule présentant une fraction amorphe dont la masse représente au moins 50%, en particulier au moins 70%, plus particulièrement au moins 90%, de la masse totale de ladite particule.

On qualifie d'amorphe un composé dans lequel les atomes ne respectent aucun ordre à moyenne et grande distance. A l'opposé, un composé cristallin est un composé dans lequel les atomes sont organisés en un réseau ordonné et respectent un ordre à moyenne et grande distance. Entre ces deux extrêmes se trouvent les composés semi-cristallins, également appelés composés partiellement amorphes.

Ainsi, on entend par « fraction amorphe » la fraction de composé sous forme amorphe ; une fraction amorphe de 0% correspond à un composé cristallin tandis qu'une fraction amorphe de 100% correspond à un composé amorphe.

On sait qu'il est intéressant, dans certains domaines de l'industrie, et notamment dans les domaines agro-alimentaire et pharmaceutique, de pouvoir disposer de particules amorphes ou de particules dont la fraction amorphe est élevée. En effet, pour des particules dont le taux d'amorphe serait inférieur à 50%, des transitions de phases ultérieures, en particulier la recristallisation d'une phase amorphe lors du stockage d'un produit comprenant lesdites particules, pourrait profondément modifier sa cinétique de dissolution ainsi que sa biodisponibilité.

Il est aujourd'hui bien établi que les formes amorphes ou semi-cristallines présentent une meilleure cinétique de dissolution, et finalement une meilleure biodisponibilité, que les formes cristallines des principes actifs très peu solubles dans les milieux aqueux.

La fraction amorphe peut être quantifiée par différentes techniques bien connues de l'homme du métier, par exemple décrites dans Threlfall, T. L. Analyst 1995, 120, p. 2435-2459 ou dans Caira, M. R. Topics in Current Chemistry 1998, 198, p 163-208, comme la diffraction aux rayons X ou la calorimétrie différentielle à balayage en mode isotherme.

Selon un mode de réalisation avantageux, la présente description divulgue également une particule dans laquelle ledit acide ou anion correspondant est choisi dans le groupe comprenant l'acide 2-hydroxy-4-méthyl-thio-butanoïque (HMTBA), la méthionine, l'acide aspartique, l'acide ascorbique, les acides alginiques, les acides pectiniques, et les anions correspondants, en particulier le 4-hydroxy-2-méthyl-thio-butanoate, le méthioninate, l'aspartate, l'ascorbate, les alginates et les pectinates.

Ledit acide ou anion correspondant est en particulier choisi dans le groupe comprenant l'acide 4-hydroxy-2-méthyl-thio-butanoïque (HMTBA), la méthionine, le 2-hydroxy-4-méthyl-thio-butanoate et le méthioninate.

Selon un mode de réalisation avantageux, la présente description concerne une particule dans laquelle ledit acide ou anion correspondant est choisi dans le groupe comprenant l'acide 2-hydroxy-4-méthyl-thio-butanoïque (HMTBA), la méthionine, l'acide aspartique, et les anions correspondants, en particulier le 2-hydroxy-4-méthyl-thio-butanoate, le méthioniate, et l'aspartate, ledit métal ou cation étant en particulier choisi parmi Ca, Mg, et les cations correspondants, plus particulièrement Ca²⁺ et Mg²⁺.

Selon un mode de réalisation avantageux, la présente description divulgue également une particule dans laquelle ledit complexe est de formule la suivante :

(Acide)ₙM (Ia)

où
M représente ledit métal,
n étant égal à 2 lorsque ledit métal est divalent et à 3 lorsque ledit métal est trivalent, ledit complexe étant en particulier un sel, plus particulièrement un sel de formule (HMTBA)₂Ca, (HMTBA)₂Mg, (HMTBA)₂Fe, (HMTBA)₂Mn, (HMTBA)₂Zn, (HMTBA)₂Cu, (HMTBA)₃Fe, (HMTBA)₃Al, (Méthionine)₂Ca, (Méthionine)₂Mg, (Méthionine)₂Fe, (Méthionine)₂Mn, (Méthionine)₂Zn, (Méthionine)₂Cu, (Méthionine)₃Fe, (Méthionine)₃Al, (acide aspartique)₂Ca, (acide aspartique)₂Mg, (acide aspartique)₂Fe, (acide aspartique)₂Mn, (acide aspartique)₂Zn, (acide aspartique)₂Cu, (acide aspartique)₃Fe, ou (acide aspartique)₃Al, encore plus particulièrement un sel de formule (HMTBA)₂Ca, (HMTBA)₂Mg, (HMTBA)₂Fe, (HMTBA)₂Mn, (HMTBA)₂Zn, (HMTBA)₂Cu, (Méthionine)₂Ca, (Méthionine)₂Mg, (Méthionine)₂Fe, (Méthionine)₂Mn, (Méthionine)₂Zn, (Méthionine)₂Cu, (acide aspartique)₂Ca, (acide aspartique)₂Mg, (acide aspartique)₂Fe, (acide aspartique)₂Mn, (acide aspartique)₂Zn ou (acide aspartique)₂Cu.

L'invention a également pour objet une particule, sous la forme d'une sphère homogène ou d'une fraction de sphère homogène, constituée essentiellement d'un complexe, notamment un sel, entre un acide ou un anion correspondant et au moins un métal ou un cation métallique correspondant, ledit acide ou anion correspondant étant l'acide 2-hydroxy-4-méthyl-thio-butanoïque (HMTBA) ou le 2-hydroxy-4-méthyl-thio-butanoate, ledit métal ou cation métallique étant divalent, choisi parmi le Ca ou le Ca²⁺, ladite particule présentant une fraction amorphe dont la masse représente au moins 50%, en particulier au moins 70%, plus particulièrement au moins 90%, de la masse totale de ladite particule, ladite particule étant substantiellement dépourvue d'acide ou d'anion non complexé et de métal ou de cation métallique non complexé, le rapport de la masse dudit acide ou anion non complexé et/ou dudit au moins un métal ou cation non complexé sur la masse totale de ladite particule étant inférieure à 20 %, et variant en particulier de 0 à 5 %, plus particulièrement de 0 à 1 %, dans laquelle ledit complexe est de formule la suivante :

(Acide)ₙM (Ia)

où M représente ledit métal, n étant égal à 2 lorsque ledit métal est divalent, ledit complexe étant en particulier un sel, plus particulièrement un sel de formule (HMTBA)₂Ca.

Ainsi, ledit acide et ledit métal peuvent être apportés dans des conditions stoéchiométriques, c'est-à-dire dans les proportions de la formule (la), pour former ledit complexe de formule (la).

Il est à noter, en particulier au niveau industriel, que les proportions d'acide et de métal sont susceptibles de s'écarter de ces conditions stoéchiométriques théoriques: en tout état de cause, l'écart éventuel est tel que, dans la particule considérée, le rapport de la masse dudit acide ou anion non complexé et/ou dudit au moins un métal ou cation non complexé sur la masse totale de ladite particule est inférieure à 20 %, et varie en particulier de 0 à 5 %, plus particulièrement de 0 à 1 %.

Selon un autre mode de réalisation avantageux, la présente description concerne une particule dans laquelle ledit complexe est choisi dans le groupe constitué des alginates de calcium et des pectinates de calcium.

Selon un mode de réalisation avantageux, la présente invention concerne une particule dans laquelle ledit complexe est de formule Ib suivante :

(Acide)₄M (Ib)

où M représente ledit métal,
ledit complexe étant en particulier de formule (HMTBA)₄Ca.

Ainsi, pour former ledit complexe de formule (Ib), en particulier (HMTBA)₄Ca, ledit acide et ledit métal sont susceptibles d'être apportés dans des conditions hyperstoéchiométriques, c'est-à-dire dans les proportions de la formule (Ib). Ces conditions sont nommées hyperstoéchiométriques, car la proportion d'acide par rapport au métal est plus importante dans la formule (Ib) que celle rencontrée dans le sel de référence de formule (la), en particulier (HMTBA)₂Ca.

Selon un mode de réalisation avantageux, la présente description concerne une particule dans laquelle ledit métal ou cation correspondant est choisi dans le groupe comprenant Li, Na, K, Mg, Be, Ca, Sr, Ba, Mn, Fe, Co, Ni, Cu, Zn, Pt, B, Al, Ga, In, et les cations correspondants, en particulier Li⁺, Na⁺, K⁺, Mg²⁺, Be²⁺, Ca²⁺, Sr²⁺, Ba²⁺, Mn²⁺, Fe²⁺, Fe³⁺, Co²⁺, Co³⁺, Ni²⁺, Ni³⁺, Cu²⁺, Zn²⁺, Pt²⁺, Al³⁺ Ga³⁺ et In³⁺.

Selon un mode de réalisation avantageux, la présente description divulgue également une particule dans laquelle ledit métal ou cation correspondant est choisi dans le groupe comprenant Mg, Be, Ca, Sr, Ba, Mn, Fe, Co, Ni, Cu, Zn, Pt, B, Al, Ga, In, en particulier Mg, Ca, Fe, Mn, Cu, Zn et les cations correspondants, en particulier Mg²⁺, Be²⁺, Ca²⁺, Sr²⁺, Ba²⁺, Mn²⁺, Fe²⁺, Fe³⁺, Co²⁺, Co³⁺, Ni²⁺, Ni³⁺, Cu²⁺, Zn²⁺, Pt²⁺, Al³⁺, Ga³⁺, In³⁺, plus particulièrement Mg²⁺, Ca²⁺, Fe²⁺, Fe³⁺, Mn²⁺, Cu²⁺ et Zn²⁺.

Selon un mode de réalisation avantageux, la présente description concerne une particule dans laquelle ledit métal ou cation correspondant est choisi dans le groupe comprenant Li, Mg, Ca, Fe, Mn, Cu et Zn et les cations correspondants, en particulier Li⁺, Mg²⁺, Ca²⁺, Fe²⁺, Fe³⁺, Mn²⁺, Cu²⁺ et Zn²⁺.

Selon un mode de réalisation avantageux, la présente description divulgue également une particule dans laquelle ledit métal ou cation correspondant est choisi dans le groupe comprenant Mg, Ca, Cu et les cations correspondants, en particulier Mg²⁺, Ca²⁺ et Cu²⁺.

Selon un mode de réalisation avantageux, la présente description concerne une particule dans laquelle ledit métal est Li ou le cation est Li⁺.

Selon un mode de réalisation avantageux, la présente description divulgue également une particule dans laquelle ledit métal est Na ou le cation est Na⁺.

Selon un mode de réalisation avantageux, la présente description concerne une particule dans laquelle ledit métal est K ou le cation est K⁺.

Selon un mode de réalisation avantageux, la présente description divulgue également une particule constituée essentiellement d'un sel entre un anion d'un acide et au moins un cation métallique.

Selon un mode de réalisation avantageux, la présente description concerne une particule dans laquelle ledit cation est choisi parmi des cations divalents et trivalents, et en particulier choisi parmi Mg²⁺, Be²⁺, Ca²⁺, Sr²⁺, Ba²⁺, Mn²⁺, Fe²⁺, Co²⁺, Ni²⁺, Cu²⁺, Zn²⁺, Pt²⁺, Fe³⁺, Co³⁺, Ni³⁺, Al³⁺, Ga³⁺, et In³⁺.

Selon un mode de réalisation avantageux, la présente description divulgue également une particule dans laquelle ledit cation est choisi parmi Li⁺, Mg²⁺, Ca²⁺, Fe²⁺, Zn²⁺, Mn²⁺, Cu²⁺ et Fe³⁺.

Selon un mode de réalisation avantageux, la présente description concerne une particule dans laquelle ledit cation est Li⁺.

Selon un mode de réalisation avantageux, la présente description divulgue également une particule dans laquelle ledit cation est Na⁺.

Selon un mode de réalisation avantageux, la présente description concerne une particule dans laquelle ledit cation est K⁺.

Selon un mode de réalisation avantageux, la présente invention concerne une particule dans laquelle ledit anion est le 2-hydroxy-4-méthyl-thio-butanoate et ledit cation est Ca²⁺.

Selon un mode de réalisation avantageux, la présente description concerne une particule dans laquelle ledit anion est le méthioninate ou le 2-hydroxy-4-méthyl-thio-butanoate et ledit cation est choisi dans le groupe comprenant Li⁺, Mg²⁺, Ca²⁺, Fe²⁺, Fe³⁺, Zn²⁺, Mn²⁺ et Cu²⁺, en particulier Li⁺, Mg²⁺ et Ca²⁺.

Selon un mode de réalisation avantageux, la présente description divulgue également une particule dans laquelle ledit anion est le méthioninate ou le 2-hydroxy-4-méthyl-thio-butanoate et ledit cation est choisi dans le groupe comprenant Mg²⁺, Ca²⁺, Fe²⁺, Fe³⁺, Zn²⁺, Mn²⁺ et Cu²⁺, en particulier Mg²⁺ et Ca²⁺.

Selon un mode de réalisation avantageux, la présente description concerne une particule dans laquelle ledit anion est le méthioninate ou le 2-hydroxy-4-méthyl-thio-butanoate et ledit cation étant Li⁺.

Selon un mode de réalisation avantageux, la présente description divulgue également une particule dans laquelle ledit anion est le méthioninate ou le 2-hydroxy-4-méthyl-thio-butanoate et ledit cation étant Na⁺.

Selon un mode de réalisation avantageux, la présente description concerne une particule dans laquelle ledit anion est le méthioninate ou le 2-hydroxy-4-méthyl-thio-butanoate et ledit cation étant K⁺.

L'invention concerne également une composition pulvérulente de particules, lesdites particules étant telles que définies précédemment.

Par « composition pulvérulente de particules », on entend un solide, dans un état fractionné, constitué de particules selon la présente invention.

Selon un mode de réalisation avantageux, la taille granulométrique desdites particules variant de 10 à 3000 µm, en particulier de 20 à 300 µm, en moyenne granulométrique [Dv(0,5)].

Par moyenne granulométrique [Dv(0,5)], on entend le diamètre granulométrique moyen, mesuré par diffraction laser, 50% des particules de ladite composition ayant un diamètre supérieur au dit diamètre moyen et 50% des particules de ladite composition ayant un diamètre inférieur au dit diamètre moyen.

Selon un mode de réalisation avantageux, ladite composition pulvérulente possède un indice d'écoulement variant de 4 à 18 [indice Flodex™].

La méthode Flodex® (Dow-Lepetit) mesure la fluidité (ou aptitude à l'écoulement) d'une poudre. Un échantillon est placé dans un cylindre lisse ayant un trou circulaire dans le fond. Le trou est obturé pendant le remplissage. Une fois que la quantité totale de poudre est introduite, le trou du fond est ouvert. Une poudre ayant une bonne fluidité s'écoule à travers un petit trou, tandis qu'une poudre ayant une mauvaise fluidité requiert un gros trou pour quitter le cylindre. L'indice de fluidité Flodex™ est égal au diamètre, en millimètres, du plus petit trou par lequel la poudre est tombée trois fois de manière consécutive.

Ladite composition pulvérulente a en outre une densité variant de 150g/L à 900 g/L, selon la norme AFNOR NF V 04-344.

Selon un mode de réalisation avantageux, la description concerne une composition pulvérulente dans laquelle, pour une partie substantielle de ladite composition, une particule de ladite partie est agglomérée avec au moins une autre particule de ladite partie.

Par « particule agglomérée avec au moins une autre particule », on entend une particule qui est juxtaposée ou fusionnée à au moins une autre particule, formant ainsi un agglomérat, c'est-à-dire un ensemble non monolithique.

Par l'expression «pour une partie substantielle de ladite composition, une particule de ladite partie est agglomérée avec au moins une autre particule de ladite partie», on entend qu'il existe une partie de la composition, dans laquelle une particule de ladite partie est agglomérée avec au moins une autre particule de ladite partie, telle que l'essentiel des plus fines particules de ladite composition soit aggloméré, collé ou fusionné à d'autres particules de la composition, de façon à ce qu'il ne reste pas plus de 10% de fines particules de la composition, inférieures à 100µm.

L'invention concerne également une composition pulvérulente de particules, lesdites particules étant telles que définies précédemment selon l'invention, la taille granulométrique desdites particules variant de 10 à 3000 µm, en particulier de 20 à 300 µm, en moyenne granulométrique [Dv(0,5)], et/ou ladite composition possédant un indice d'écoulement variant de 4 à 18 [indice flodex], et/ou ladite composition étant telle que l'essentiel des plus fines particules de la composition soit aggloméré, collé ou fusionné à d'autres particules de la composition, de façon à ce qu'il ne reste pas plus de 10 % de fines particules de la composition, inférieures à 100 µm.

L'invention concerne également l'utilisation d'une composition pulvérulente telle que décrite précédemment, en alimentation animale.

L'invention concerne également un procédé de préparation d'une composition pulvérulente de particules constituées essentiellement d'un complexe, notamment un sel, entre un acide ou un anion correspondant et au moins un métal ou un cation métallique correspondant, ledit procédé comprenant une étape de mise en contact dudit acide avec une source minérale dudit métal ou cation correspondant dans une tour de séchage par atomisation pour obtenir la formation dudit complexe, notamment dudit sel, et initier sa précipitation.

De manière surprenante, les auteurs ont remarqué que la cinétique de ladite précipitation était parfaitement compatible avec un procédé de séchage par atomisation malgré une durée de mise en contact ne dépassant pas quelques minutes, même généralement moins d'une minute.

Les auteurs ont également eu la surprise de constater qu'un procédé selon l'invention autorisait un extrait sec du mélange réactionnel élevé, sans bouchage, ledit mélange réactionnel étant obtenu par ladite mise en contact dudit acide avec une source minérale dudit métal ou cation correspondant.

Le procédé selon l'invention permet d'obtenir ladite composition pulvérulente après la précipitation dudit complexe et le séchage de ladite composition pulvérulente, et ce, malgré l'accroissement exponentiel de la viscosité observé lors de ladite précipitation.

De plus, à l'issue d'un procédé selon l'invention, la composition pulvérulente obtenue est directement manipulable, à la bonne granulométrie, où la possibilité de recyclage, dans le cadre de la mise en forme de la composition pulvérulente telle qu'elle est effectuée par l'homme de l'art dans une tour multiple effet, n'altère en rien les capacités de production ni la qualité du produit issu du procédé, ce qui en fait un procédé extrêmement robuste et productif.

Par composition directement manipulable, on entend une composition dont l'écoulement permet la manipulation, le transfert, le chargement ou le déchargement.

Enfin, le séchage par atomisation étant extrêmement rapide, aucune réaction de brunissement n'apparait comme souvent observé sur des produits déjà commercialisés, ou déjà obtenus par d'autres procédés.

Par séchage par atomisation selon l'invention, on entend un procédé de séchage du mélange réactionnel obtenu par ladite mise en contact dudit acide avec une source minérale dudit métal ou cation correspondant, en pulvérisant ledit mélange dans un courant d'air chaud à l'aide de dispositifs de pulvérisation tels que des buses, des turbines ou des disques rotatifs. Le séchage et le transfert de l'eau se font par entraînement dans l'air du fait de la différence de pression de vapeur entre la gouttelette formée et l'air à la périphérie de la gouttelette.

Il est à noter qu'un séchage par atomisation d'un mélange réactionnel comme rencontré dans la présente invention est appelé séchage par atomisation réactive.

Divers dispositifs permettent de réaliser le séchage par atomisation réactive selon l'invention. A titre d'exemple de dispositif de séchage par atomisation, on peut citer des tours de séchage simple effet, toll form, à lit d'air fluidisé interne ou externe de type tour multiple effet, bien connues de l'homme du métier.

Par exemple, dans une tour simple effet de 10Kg / H de capacité évaporatoire, un mélange stoechiométrique d'un acide, par exemple le HMTBA, en milieu aqueux et une source métalique d'un métal ou cation, par exemple de la chaux en suspension dans l'eau, dans des débits respectifs de 2 à 7 kg/H, avec des température d'entrée de 180 à 100°C et des températures de sortie de 150 à 65°C, permet d'obtenir 1 à 10 kg/ H de composition pulvérulente selon l'invention.

Ledit dispositif permettant de réaliser le séchage par atomisation réactive selon l'invention comprend éventuellement un dispositif de pulvérisation complémentaire d'agent antiagglomérant, par exemple via un doseur de poudre.

La présente demande concerne également un procédé de préparation d'une composition pulvérulente de particules telles que décrites précédemment, constituées essentiellement d'un complexe, notamment un sel, entre un acide ou un anion correspondant et au moins un métal ou un cation métallique correspondant, ledit procédé comprenant une étape de mise en contact dudit acide avec une source minérale dudit métal ou cation correspondant dans une tour de séchage par atomisation pour obtenir la formation dudit complexe, notamment dudit sel, et initier sa précipitation,
ledit acide ou anion correspondant étant choisi dans le groupe comprenant l'acide 2-hydroxy-4-méthyl-thio-butanoïque (HMTBA), la méthionine, l'acide aspartique, les acides alginiques, les acides pectiniques, et les anions correspondants, en particulier le 2-hydroxy-4-méthyl-thio-butanoate, le méthioniate, l'aspartate, les alginates et les pectinates,
ledit métal ou cation métallique étant divalent ou trivalent,
ladite particule présentant une fraction amorphe dont la masse représente au moins 50%, en particulier au moins 70%, plus particulièrement au moins 90%, de la masse totale de ladite particule.

L'invention concerne également un procédé de préparation d'une composition pulvérulente de particules, lesdites particules étant telles que définies précédemment selon l'invention, constituées essentiellement d'un complexe, notamment un sel, entre un acide ou un anion correspondant et au moins un métal ou un cation métallique correspondant, ledit procédé comprenant une étape de mise en contact dudit acide avec une source minérale dudit métal ou cation correspondant dans une tour de séchage par atomisation pour obtenir la formation dudit complexe, notamment dudit sel, et initier sa précipitation, ledit acide ou anion correspondant étant l'acide 2-hydroxy-4-méthyl-thio-butanoïque (HMTBA), ou le 2-hydroxy-4-méthyl-thio-butanoate, ledit métal ou cation métallique étant divalent, choisi parmi le Ca ou le Ca²⁺, ladite particule présentant une fraction amorphe dont la masse représente au moins 50%, en particulier au moins 70%, plus particulièrement au moins 90%, de la masse totale de ladite particule.

Selon un mode de réalisation avantageux, un procédé selon l'invention comprend une étape de pulvérisation au cours de laquelle la précipitation dudit complexe, notamment dudit sel, se poursuit.

Selon un mode de réalisation particulièrement avantageux, les étapes de mise en contact et de pulvérisation sont réalisées à l'aide du même dispositif, en particulier un dispositif comprenant un organe de pulvérisation tournant, la mise en contact étant immédiatement suivie de la pulvérisation.

Par « la mise en contact étant immédiatement suivie de la pulvérisation », on entend qu'au sein d'un même dispositif, un moyen de mise en contact est directement lié à un moyen de pulvérisation.

Les dispositifs permettant de réaliser en leur sein l'étape de mise en contact, suivie immédiatement de l'étape de pulvérisation, sont en particulier mais de façon non limitative, des turbines ou des disques rotatifs tels que décrits dans la demande de brevet EP 2257380; la mise en contact a alors par exemple lieu dans une chambre de mélange d'une turbine, puis le complexe ainsi formé est, immédiatement après la mise en contact, pulvérisé à l'aide de ladite turbine.

Selon un autre mode de réalisation avantageux, un procédé selon l'invention comprend une étape de séchage par atomisation dudit complexe, notamment dudit sel, au cours de laquelle la précipitation dudit complexe, notamment dudit sel, se poursuit jusqu'à solidification complète de la particule.

Selon un autre mode de réalisation avantageux, un procédé selon l'invention comprend les étapes suivantes :
- mise en contact dudit acide avec une source minérale dudit métal ou cation correspondant dans une tour de séchage par atomisation pour obtenir la formation dudit complexe, notamment dudit sel, et initier sa précipitation ;
- pulvérisation dudit complexe, notamment dudit sel, en cours de précipitation, au cours de laquelle ladite précipitation se poursuit, pour obtenir un ensemble de particules pulvérisées ;
- séchage par atomisation dudit ensemble de particules pulvérisées, au cours de laquelle ladite précipitation se poursuit jusqu'à solidification complète de la particule, pour obtenir une composition pulvérulente, stable ;
- récupération de ladite composition pulvérulente.

Par composition pulvérulente stable, on entend une composition pulvérulente qui ne motte pas dans les conditions de stockage normales, telles qu'une température inférieure à 20°C et une humidité relative inférieure à 70%.

Une composition pulvérulente stable ne nécessite pas de broyage subséquent.

Selon un mode de réalisation particulièrement avantageux, un procédé selon l'invention comprend les étapes suivantes :
- mise en contact dudit acide avec une source minérale dudit métal ou cation correspondant dans une tour de séchage par atomisation, à l'aide d'un dispositif permettant ladite mise en contact, pour obtenir la formation dudit complexe, notamment dudit sel, et initier sa précipitation ;
- pulvérisation dudit complexe, notamment dudit sel, en cours de précipitation, au cours de laquelle ladite précipitation se poursuit, à l'aide d'un dispositif de pulvérisation, pour obtenir un ensemble de particules pulvérisées, ladite pulvérisation étant immédiatement subséquente à la mise en contact, ledit dispositif de pulvérisation étant identique à celui permettant la mise en contact, en particulier un dispositif comprenant un organe de pulvérisation tournant ;
- séchage par atomisation dudit ensemble de particules pulvérisées, au cours de laquelle ladite précipitation se poursuit jusqu'à solidification complète de la particule, pour obtenir une composition pulvérulente, stable ;
- récupération de ladite composition pulvérulente.

Selon un autre mode de réalisation avantageux, ladite mise en contact se fait par mélange d'un milieu aqueux, en particulier une solution aqueuse, contenant ledit acide et d'un milieu aqueux, en particulier une solution ou une suspension aqueuse contenant ledit métal ou cation.

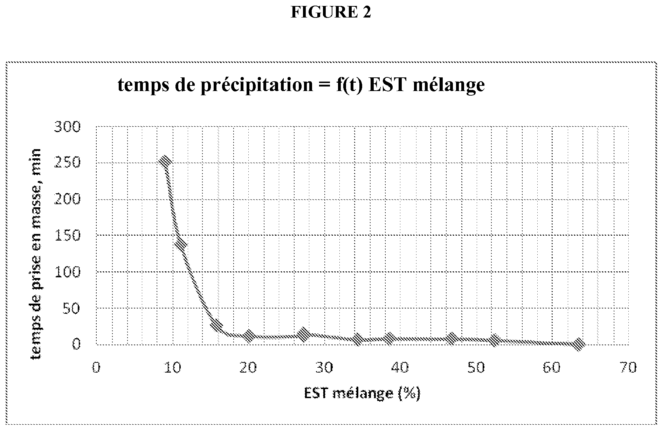

Un procédé selon l'invention permet ainsi d'obtenir ladite composition pulvérulente lorsque la précipitation du complexe est initiée lors de la mise en contact dudit acide avec une source minérale dudit métal ou cation correspondant, c'est-à-dire lorsque le milieu aqueux contenant ledit acide et ledit métal ou cation ont des concentrations respectives en acide et en métal élevées, à laquelle la précipitation dudit complexe est obtenue. Pour permettre l'obtention de la composition pulvérulente en effectuant séparément la mise en contact dudit acide et dudit métal ou cation, puis la mise sous forme de poudre du sel formé, il faudrait atteindre des concentrations en acide et en métal très faibles comme le montre la courbe de temps de précipitation en fonction de la matière sèche du mélange représentée à la figure 2, ce qui conduirait à une mise sous forme de poudre du sel formé dans des conditions économiques très défavorables.

Ainsi, un procédé selon l'invention permet de travailler à des concentrations en acide élevée, plus élevées que dans les procédés dans lesquels la précipitation dudit mélange est indésirable et donc évitée en utilisant notamment des milieux aqueux contenant ledit acide et/ou des milieu aqueux contenant ledit métal ou cation à des concentrations relativement basses.

L'acide et ladite source du métal ou cation, en particulier lesdits milieux aqueux contenant respectivement ledit acide et ledit métal ou cation, sont alimentés dans lesdits dispositifs de pulvérisation afin de constituer ledit mélange réactionnel et d'y produire après séchage ladite composition pulvérulente. Il appartient bien entendu aux connaissances générales de l'homme du métier d'adapter les caractéristiques desdits dispositifs.

Le choix du dispositif de pulvérisation sera notamment conçu pour assurer de manière concomitante ou non le mélange desdits milieux aqueux et ladite pulvérisation.

Les conditions optimales de temps, de température, d'énergie nécessaire pour ladite pulvérisation dépendent de la nature chimique des réactifs que sont ledit acide et ladite source du métal ou cation et la nature chimique dudit complexe recherché, et seront déterminés au cas par cas par l'homme du métier.

Les débits d'alimentations desdits deux réactifs, acide et source du métal ou cation, sont régulés en fonction de leur nature chimique respective et du type de complexe recherché.

Les dispositifs de pulvérisation sont préférentiellement, mais de façon non limitative, des buses de pulvérisation haute pression, monofluides ou bifluides, à mélange externe ou interne, dans lesquelles le fluide vecteur peut être de l'air, un gaz neutre ou de la vapeur ou un mélange de ces fluides ; des turbines, des bols inversés ou des disques rotatifs tels que décrits dans la demande de brevet EP 2257380 peuvent être employés.

Les dispositifs de pulvérisation, composés de la ligne d'alimentation de la pulvérisation et de l'organe de pulvérisation pourront préférentiellement être conçues pour assurer un temps de séjour minimal nécessaire à la formation du complexe et à l'initiation de la précipitation dudit complexe.

Pour accroitre l'efficacité de la mise en contact, il peut être avantageux d'ajouter une chambre de mélange avant ou en cours de pulvérisation pour assurer une durée de mise en contact, avant pulvérisation, relativement courte, de quelques minutes au maximum.

Selon un mode de réalisation avantageux, ladite mise en contact dure de 400 ms à 5 minutes, en particulier de 5 à 30 secondes.

Selon un mode de réalisation avantageux, ladite mise en contact dure de 100 ms à 5 minutes, en particulier de 100 ms à 30 s.

Selon un autre mode de réalisation avantageux, la température lors de ladite mise en contact et jusqu'à ladite pulvérisation varie de la température ambiante à 150°C, de préférence entre 40 et 120°C, de manière encore plus préférée entre 60 et 95°C.

Selon un autre mode de réalisation avantageux, la pression lors de ladite mise en contact et jusqu'à ladite pulvérisation varie de la pression atmosphérique à 600 bars.

La chambre de mélange peut-être équipée d'un dispositif adapté de mélangeur interne.

Selon un mode de réalisation avantageux, ladite mise en contact est réalisée dans un dispositif tel que les mélangeurs statiques ou dynamiques, en particulier les malaxeurs, les extrudeurs, et les mélangeurs sans pièce interne, comme les mélangeurs à ultrason.

L'ensemble du dispositif de séchage par atomisation, en particulier les dispositifs de pulvérisation sus décrits, permet d'optimiser l'obtention de la composition pulvérulente avec des taux de transformation des réactifs très élevés.

La composition pulvérulente finale, sous forme d'une poudre sèche à granulométrie contrôlée, est ainsi récupérée directement en un seul passage, sans nécessité d'une quelconque manipulation complémentaire.

Afin de disposer d'une composition pulvérulente encore plus homogène en granulométrie, le dispositif de séchage pourra être complété par l'injection des fines particules issues du procédé directement dans la zone de pulvérisation ou par l'utilisation des dispositifs de pulvérisation de type disques rotatifs tels que décrits dans le demande de brevet EP 2257380 pour maîtriser de manière plus efficiente l'aérosol généré.

L'emploi d'une tour de séchage dans laquelle le temps de séjour est le plus élevé possible, de quelques secondes à plusieurs minutes, est préféré. Le procédé est avantageusement réalisé dans une tour de type multiple effet ou de type toll form très haute, avec un lit d'air fluidisé intégré en bas d'équipement et sécheur d'air à lit fluidisé externe suivant l'application du produit final, la teneur en eau attendue et en intégrant la cinétique de précipitation du complexe obtenu.

Selon un autre mode de réalisation avantageux, un procédé selon l'invention comprend une étape supplémentaire d'agglomération.

Ainsi, une agglomération peut être réalisée par pulvérisation d'eau avec une buse de pulvérisation, sur un lit de composition pulvérulente par exemple de 500g sur un lit d'air fluidisé de type Glatt GPCG1, avec un débit d'air de fluidisation de 150 à 300 m3/H, un débit d'eau de pulvérisation de 2 à 20 g/mn avec une température d'entrée de 40 à 120°C et une température de sortie de 25 à 100°C.

Selon un autre mode de réalisation avantageux, ladite tour de séchage par atomisation est une tour de séchage par atomisation multiple effet, ladite étape séchage comprenant également une agglomération.

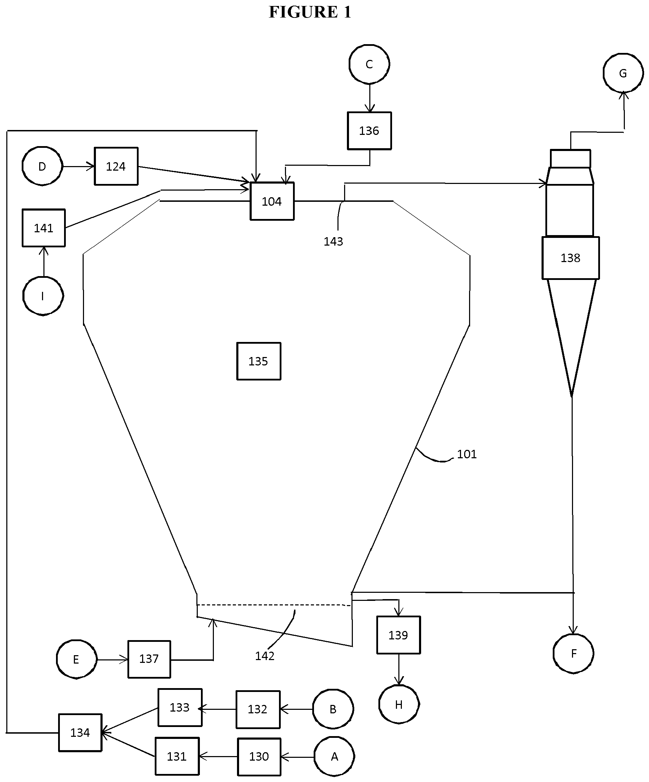

Ainsi, le séchage et l'agglomération peuvent être avantageusement réalisés sur une tour de type MSD de 100 à 400 Kg / H de capacité évaporatoire, par pulvérisation des dits milieux aqueux dans la tour de séchage comprenant un lit statique en bas de tour selon la figure 1 avec des débits de pulvérisation de 50 à 500 kg/H, des températures d'entrée de 100 à 250°C et des températures de sortie de 40 à 150°C.

Selon un autre mode de réalisation avantageux, ladite mise en contact dudit acide avec une source minérale dudit métal ou cation correspondant est réalisée en continu.

Selon un autre mode de réalisation avantageux, ledit acide ou anion correspondant est choisi dans le groupe comprenant l'acide 2-hydroxy-4-méthyl-thio-butanoïque (HMTBA), la méthionine, l'acide aspartique, l'acide ascorbique, les acides alginiques, les acides pectiniques, et les anions correspondants, en particulier le 2-hydroxy-4-méthyl-thio-butanoate, le méthioninate, l'aspartate, l'ascorbate, les alginates et les pectinates.

Selon un mode de réalisation avantageux, la présente demande concerne un procédé tel que décrit précédemment, dans lequel ledit acide ou anion correspondant est choisi dans le groupe comprenant l'acide 2-hydroxy-4-méthyl-thio-butanoïque (HMTBA), la méthionine, l'acide aspartique, et les anions correspondants, en particulier le 2-hydroxy-4-méthyl-thio-butanoate, le méthioniate, et l'aspartate, ledit métal ou cation étant en particulier choisi parmi Ca, Mg, et les cations correspondants, plus particulièrement Ca²⁺ et Mg²⁺.

Selon un mode de réalisation avantageux, la demande divulgue un procédé tel que décrit précédemment, dans lequel ledit complexe est de formule la suivante :

(Acide)ₙM (la)

où
M représente ledit métal,
n étant égal à 2 lorsque ledit métal est divalent et à 3 lorsque ledit métal est trivalent, ledit complexe étant en particulier un sel, plus particulièrement un sel de formule (HMTBA)₂Ca, (HMTBA)₂Mg, (HMTBA)₂Fe, (HMTBA)₂Mn, (HMTBA)₂Zn, (HMTBA)₂Cu, (HMTBA)₃Fe, (HMTBA)₃Al, (Méthionine)₂Ca, (Méthionine)₂Mg, (Méthionine)₂Fe, (Méthionine)₂Mn, (Méthionine)₂Zn, (Méthionine)₂Cu, (Méthionine)₃Fe, (Méthionine)₃Al, (acide aspartique)₂Ca, (acide aspartique)₂Mg, (acide aspartique)₂Fe, (acide aspartique)₂Mn, (acide aspartique)₂Zn, (acide aspartique)₂Cu, (acide aspartique)₃Fe, ou (acide aspartique)₃Al, encore plus particulièrement un sel de formule (HMTBA)₂Ca, (HMTBA)₂Mg, (HMTBA)₂Fe, (HMTBA)₂Mn, (HMTBA)₂Zn, (HMTBA)₂Cu, (Méthionine)₂Ca, (Méthionine)₂Mg, (Méthionine)₂Fe, (Méthionine)₂Mn, (Méthionine)₂Zn, (Méthionine)₂Cu, (acide aspartique)₂Ca, (acide aspartique)₂Mg, (acide aspartique)₂Fe, (acide aspartique)₂Mn, (acide aspartique)₂Zn ou (acide aspartique)₂Cu.

Selon un autre mode de réalisation avantageux, la présente demande concerne un procédé dans lequel ledit complexe est choisi dans le groupe constitué des alginates de calcium et des pectinates de calcium.

Selon un mode de réalisation avantageux, l'invention concerne un procédé tel que décrit précédemment, dans lequel ledit complexe est de formule Ib suivante :

(Acide)₄M (Ib)

où M représente ledit métal,
ledit complexe étant en particulier de formule (HMTBA)₄Ca.

La présente invention concerne également un procédé tel que défini précédemment selon l'invention, dans lequel ledit complexe est de formule la suivante :

(Acide)ₙM (Ia)

où M représente ledit métal, ledit complexe étant en particulier un sel, plus particulièrement un sel de formule (HMTBA)₂Ca,.

Selon un autre mode de réalisation avantageux, ledit métal ou cation correspondant est choisi dans le groupe comprenant Li, Na, K, Mg, Be, Ca, Sr, Ba, Mn, Fe, Co, Ni, Cu, Zn, Pt, B, Al, Ga, In, et les cations correspondants, en particulier Li⁺, Na⁺, K⁺, Mg²⁺, Be²⁺, Ca²⁺, Sr²⁺, Ba²⁺, Mn²⁺, Fe²⁺, Fe³⁺, Co²⁺, Co³⁺, Ni²⁺, Ni³⁺, Cu²⁺, Zn²⁺, Pt²⁺, Al³⁺, Ga³⁺ et In³⁺..

Selon un mode de réalisation particulièrement avantageux, ledit métal ou cation correspondant est choisi dans le groupe comprenant comprenant Li, Mg, Ca, Fe, Mn, Cu et Zn et les cations correspondants, en particulier Li⁺, Mg²⁺, Ca²⁺, Fe²⁺, Fe³⁺, Mn²⁺, Cu²⁺ et Zn²⁺.

Selon un mode de réalisation avantageux, la présente demande concerne un procédé tel que décrit précédemment, dans lequel ledit métal ou cation correspondant est choisi dans le groupe comprenant Mg, Be, Ca, Sr, Ba, Mn, Fe, Co, Ni, Cu, Zn, Pt, B, Al, Ga, In, en particulier Mg, Ca, Fe, Mn, Cu, Zn et les cations correspondants, en particulier Mg²⁺, Be²⁺, Ca²⁺, Sr²⁺, Ba²⁺, Mn²⁺, Fe²⁺, Fe³⁺, Co²⁺, Co³⁺, Ni²⁺, Ni³⁺, Cu²⁺, Zn²⁺, Pt²⁺, Al³⁺, Ga³⁺, In³⁺, plus particulièrement Mg²⁺, Ca²⁺, Fe²⁺, Fe³⁺, Mn²⁺, Cu²⁺ et Zn²⁺.

Selon un mode de réalisation avantageux, la description divulgue un procédé tel que décrit précédemment, dans lequel ledit métal ou cation correspondant est choisi dans le groupe comprenant Mg, Ca, Cu et les cations correspondants, en particulier Mg²⁺, Ca²⁺ et Cu²⁺.

Selon un mode de réalisation avantageux, la présente demande concerne un procédé dans lequel ledit métal est Li ou le cation est Li⁺.

Selon un mode de réalisation avantageux, la présente description divulgue un procédé dans lequel ledit métal est Na ou le cation est Na⁺.

Selon un mode de réalisation avantageux, la présente demande concerne un procédé dans lequel ledit métal est K ou le cation est K⁺.

Selon un mode de réalisation particulièrement avantageux, lesdites particules sont constituées essentiellement d'un sel entre un anion d'un acide et au moins un cation métallique.

Selon un autre mode de réalisation avantageux, ledit cation est choisi parmi des cations divalents et trivalents, et en particulier choisi parmi Mg²⁺, Be²⁺, Ca²⁺, Sr²⁺, Ba²⁺, Mn²⁺, Fe²⁺, Co²⁺, Ni²⁺, Cu²⁺, Zn²⁺, Pt²⁺, Fe³⁺, Co³⁺, Ni³⁺, Al³⁺, Ga³⁺, et In³⁺.

Selon un mode de réalisation particulièrement avantageux, ledit cation est choisi parmi Li⁺, Mg²⁺, Ca²⁺, Fe²⁺, Zn²⁺, Mn²⁺, Cu²⁺ et Fe³⁺.

Selon un mode de réalisation avantageux, la présente description divulgue un procédé dans lequel ledit cation est Li⁺.

Selon un mode de réalisation avantageux, la présente demande concerne un procédé dans lequel ledit cation est Na⁺.

Selon un mode de réalisation avantageux, la présente description divulgue un procédé dans lequel ledit cation est K⁺.

Selon un mode de réalisation avantageux, la présente demande concerne un procédé dans lequel ledit anion est le méthioninate ou le 2-hydroxy-4-méthyl-thio-butanoate et ledit cation est choisi dans le groupe comprenant Mg²⁺, Ca²⁺, Fe²⁺, Fe³⁺, Zn²⁺, Mn²⁺ et Cu²⁺, en particulier Mg²⁺ et Ca²⁺.

Selon un mode de réalisation avantageux, la présente description divulgue un procédé dans lequel ledit anion est le méthioninate ou le 2-hydroxy-4-méthyl-thio-butanoate et ledit cation étant Li⁺.

Selon un mode de réalisation avantageux, la présente demande concerne un procédé dans lequel ledit anion est le méthioninate ou le 2-hydroxy-4-méthyl-thio-butanoate et ledit cation étant Na⁺.

Selon un mode de réalisation avantageux, la présente description divulgue un procédé dans lequel ledit anion est le méthioninate ou le 2-hydroxy-4-méthyl-thio-butanoate et ledit cation étant K⁺.

Selon un autre mode de réalisation avantageux, ladite source minérale dudit métal ou cation est choisie parmi les hydroxydes métalliques, les laits d'hydroxyde métallique, les oxydes métalliques et les carbonates métalliques correspondants.

Selon un autre mode de réalisation avantageux, ladite source minérale dudit métal ou cation est d'origine naturelle.

Selon un autre mode de réalisation particulièrement avantageux, ledit anion est le 2-hydroxy-4-méthyl-thio-butanoate et ledit cation est Ca²⁺, la source de Ca²⁺ étant choisie parmi la chaux, le lait de chaux, la chaux éteinte, l'hydrogénocarbonate de calcium et le carbonate de calcium.

Selon un autre mode de réalisation avantageux, ladite source de Ca²⁺ est le Ca(OH)₂.

Selon un autre mode de réalisation avantageux, ledit anion est le méthioninate ou le 2-hydroxy-4-méthyl-thio-butanoate et ledit cation est choisi dans le groupe comprenant Li⁺, Mg²⁺, Ca²⁺, Fe²⁺, Fe³⁺, Zn²⁺, Mn²⁺ et Cu²⁺, la source dudit cation étant choisie parmi l'oxyde, l'hydroxyde, une solution aqueuse d'hydroxyde et le carbonate dudit cation.

La présente demande concerne également une particule susceptible d'être obtenue par le procédé décrit précédemment.

La présente demande concerne également un ensemble de particules susceptible d'être obtenu par le procédé décrit précédemment.

La présente demande concerne également des agglomérats susceptibles d'être obtenus par le procédé décrit précédemment.

### DESCRIPTION DES FIGURES

La Figure 1 est un schéma de principe d'un procédé selon l'invention, mis en oeuvre dans une tour à multiples effets.
   Un milieu aqueux contenant un acide, symbolisé par le cercle A, traverse éventuellement un réchauffeur 130 et alimente par une pompe 131 le dispositif de mise en contact 134. Un milieu aqueux contenant un métal ou cation métallique symbolisé par le cercle B traverse éventuellement un réchauffeur 132 et alimente par une pompe 133 le dispositif de mise en contact 134. La phase aqueuse résultant du mélange entre le milieu aqueux A et le milieu aqueux B, est pulvérisée dans la tour d'atomisation via le dispositif de pulvérisation 104 destiné à la production d'aérosols monodisperses ou polydisperses.
   Le cercle C représente un dispositif de pulvérisation complémentaire d'agent antiagglomérant via un doseur de poudre 136, si nécessaire.
   Le cercle D représente l'introduction du gaz vecteur chaud en version séchage par atomisation, via le ventilateur 124.
   Le cercle E représente l'introduction du gaz vecteur secondaire, pour le séchage ou /et le refroidissement final de la composition finale stabilisée obtenue, solide ou en cours de solidification via un ventilateur 137.
   Un cyclone 138 sépare tout ou partie du produit final F c'est-à-dire la composition pulvérulente, que l'on récupère, et le gaz vecteur G que l'on évacue.
   Il peut également être prévu un lit fluidisé vibré externe 139 permettant la récupération de tout ou partie du produit final H, c'est-à-dire la composition pulvérulente, par le bas de la tour.
   L'introduction de l'air secondaire E a lieu à travers un fond perméable 142 de la tour 135 pour mettre la matière pulvérulente sous forme de lit fluidisé. L'air usé est évacué par un orifice 143 prévu à travers la paroi supérieure de l'enceinte 101. Dans cet exemple, l'air usé passe ensuite par le cyclone 138 qui produit d'un côté des particules de produit F et de l'autre de l'air à évacuer G. La majeure partie des particules est recueillie juste au-dessus de la paroi perméable 142. La figure 1 illustre que les particules sont collectées soit directement en F, soit par l'intermédiaire du lit fluidisé externe 139 en H lorsqu'il en est prévu un.
   Il peut en outre être prévu l'addition, représentée par le cercle I, en zone de pulvérisation, d'une substance en poudre, en particulier des fines particules de la composition pulvérulente récupérées en sortie du cyclone 138, produit F, ou de l'installation, injectée au moyen du dispositif 141 constitué principalement d'un doseur de poudre.
La figure 2 est un graphique représentant de temps de précipitation (en minutes) d'un mélange entre un lait de chaux et l'HMTBA, en fonction de l'extrait sec total dudit mélange (en pourcents).
La figure 3 est un graphique relatif à l'échantillon « R », représentant le flux de chaleur en fonction de la température, mesuré par calorimétrie différentielle à balayage en mode isotherme (en DSC - differential scanning calorimetry).
   On note un pic de fusion à 103°C lors de la montée en Température, et un pic de cristallisation à 92°C (début du pic à 102°C) lors de la descente en température.
La figure 4 est un graphique relatif à l'échantillon « T7 », représentant le flux de chaleur en fonction de la température, mesuré par calorimétrie différentielle à balayage en mode isotherme (en DSC - differential scanning calorimetry).
   On note un pic endothermique de très faible amplitude à 82°C lors de la montée en température, suivi d'une plage endothermique étendue non caractéristique de pics de fusion. On n'observe pas de phénomène thermique lors de la descente en température, donc il y a absence de recristallisation du produit.

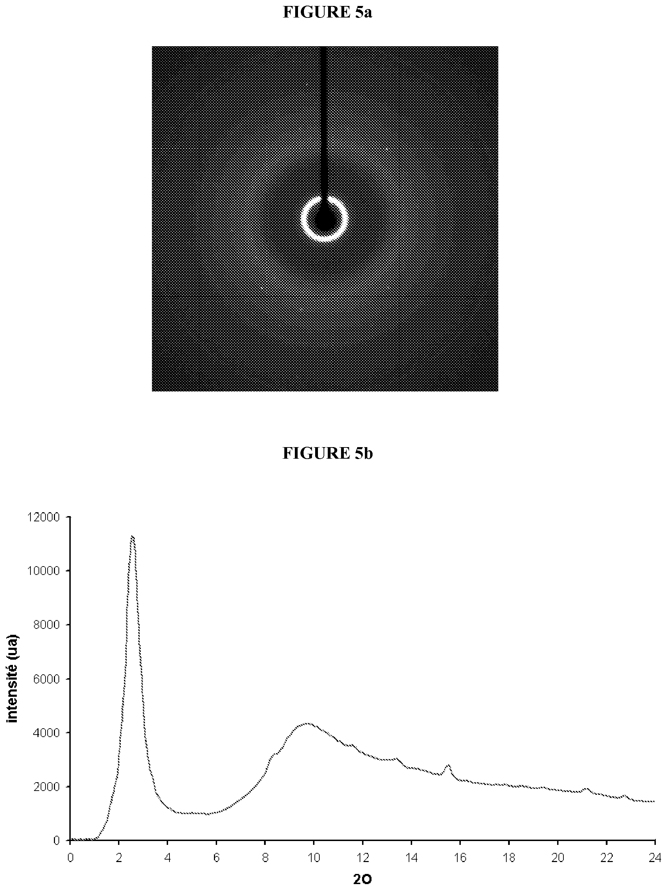

La figure 5a représente le cliché de diffraction 2D obtenu pour l'échantillon « T7 ».
La figure 5b représente le diagramme de poudre obtenu pour l'échantillon « T7 ».

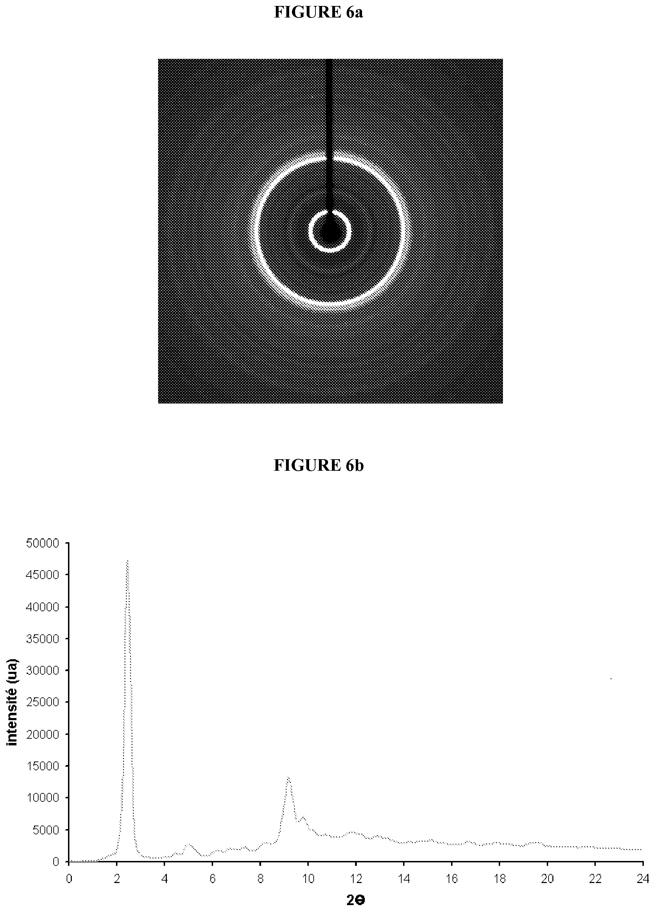

La figure 6a représente le cliché de diffraction 2D obtenu pour l'échantillon « R ».
La figure 6b représente le diagramme de poudre obtenu pour l'échantillon « R ».

L'analyse des échantillons T7 et R par diffraction aux rayons X a été effectuée à l'aide d'un diffractomètre Bruker APEX-II Quasar équipé d'une microsource au molybdène (λ = 0.71073 Ǻ).

Chaque échantillon a été préalablement broyé à l'aide d'un mortier pendant deux minutes.

Chacune des poudres ainsi obtenue est ensuite transférée dans un capillaire de 0,5 mm de diamètre transparent aux rayons X, puis montée sur un goniomètre.

Les caractéristiques de l'enregistrement sont parfaitement identiques pour chaque échantillon, à savoir :
- distance du détecteur : 80mm
- durée d'enregistrement : 999s
- rotation uniforme de l'échantillon selon l'axe Phi de 359° durant l'acquisition
- position des anges Chi, Kappa et Omega = 0°
- température d'enregistrement 280 K

Les clichés de diffraction 2D obtenus pour chacun des échantillons correspondent aux figures 5a et 6a.

Le regroupement angulaire de ces images bidimensionnelles a ensuite été réalisé à l'aide du logiciel XRD2DScan 4.1.1, après avoir soustrait à chaque image celle du bruit de fond enregistré dans les mêmes conditions, pour obtenir les courbes représentées aux figures 5b et 6b.

Les exemples 1 à 9 qui suivent illustrent l'invention.

### EXEMPLES

### Exemple 1 : Préparation du sel (HMTBA)₂Ca

Un lait de chaux préparé à 30% de matière sèche, est mélangé en continu dans une conduite contenant un mélangeur statique avec une solution d'HMTBA à 88% de matière sèche dans le ratio 22,2% de chaux (calculé sur la matière sèche engagée) et 77,8% d'HMTBA (calculé sur la matière sèche engagée).

Le temps de mise en contact est de 7 secondes.

Le mélange réactionnel est alors pulvérisé à l'aide d'une buse selon la connaissance de l'homme de l'art, dans une tour d'atomisation simple effet avec une température d'entrée de 140°C et une température de sortie de 79°C.

Le produit est ensuite repris en sécheur à lit d'air fluidisé pour obtenir une poudre agglomérée pour simuler une tour d'atomisation multiple effet.

Le produit obtenu a une teneur en HMTBA de 81,4 %, en Ca²⁺ de 11,8%, et une humidité de 1,3%. La granulométrie moyenne est de 240 µm et la densité de 300 g/L.

### Exemple 2 : Autre préparation du sel (HMTBA)₂Ca

Un lait de chaux préparé à 30% de matière sèche, est mélangé en continu dans une conduite contenant un mélangeur statique à 138 kg/H avec une solution d'HMTBA à 88% de matière sèche à 154 Kg / H pour donner un mélange réactionnel de 60% de matière sèche.

Le temps de mise en contact est de 15 secondes.

Le mélange réactionnel est pulvérisé à l'aide d'une buse selon la connaissance de l'homme de l'art, dans une tour d'atomisation simple effet avec une température d'entrée de 185°C et une température de sortie de 128°C

Le produit obtenu contient 84,9% d'HMTBA, 12,0% de Ca²⁺ et 0,5% d'eau.

La granulométrie moyenne est de 156µm (Dv(0.5) en granulométrie laser), la densité est de 170 g/l.

### Exemple 3 : Préparation d'un sel de Mg de HMTBA (comparatif)

Une suspension d'hydroxyde de magnésium à 20% de matière sèche est mélangée en continu dans une conduite contenant un mélangeur statique à 2,9 kg/H, avec une solution d'HMTBA à 70% de matière sèche à 4,3 Kg / H.

Le temps de mise en contact lors de l'atomisation réactive est de 7 secondes.

Le mélange réactionnel est alors pulvérisé à l'aide d'une buse selon la connaissance de l'homme de l'art dans une tour d'atomisation simple effet avec une température d'entrée de 140°C et une température de sortie de 76°C.

Le produit obtenu a une teneur en HMTBA de 91,2%, une teneur en Mg²⁺ de 7,4% et une humidité de 1,4%.

La granulométrie moyenne est de 7 µm et la densité de 310 g/L.

### Exemple 4 : Préparation d'un sel de Li de HMTBA (comparatif)

Un lait d'hydroxyde de lithium préparé à 10% de matière sèche, est mélangé en continu dans une conduite contenant un mélangeur statique à 3,6 kg/H avec une solution d'HMTBA à 70% de matière sèche à 4,0 kg/H.

Le temps de mise en contact est de 7 secondes.

Le mélange réactionnel est alors pulvérisé à l'aide d'une buse selon la connaissance de l'homme de l'art dans une tour d'atomisation avec une température d'entrée de 160°C et une température de sortie de 70°C.

Le produit obtenu contient 89,2 % de HMTBA, 4,2% de Li⁺ et 6,6 % d'eau.

La granulométrie moyenne est de 5µm, la densité est de 360 g/L.

### Exemple 5 : Préparation du sel (HMTBA)₂Ca à l'aide d'une turbine d'atomisation

Un lait de chaux préparé à 20% de matière sèche, est mélangé en continu dans une turbine d'atomisation (de type NIRO Atomiseur) à 3,9 kg/H avec une solution d'HMTBA à 70% de matière sèche à 3,4 Kg/H.

Le temps de mise en contact est de 120 millisecondes.

Le produit est alors atomisé dans une tour d'atomisation simple effet avec une température d'entrée de 140°C et une température de sortie de 90°C.

Le produit obtenu a une teneur en HMTBA de 85,0 %, en Ca²⁺ de 10,7%, et une humidité de 1,3%.

La granulométrie moyenne est de 43 µm et la densité de 380 g/L.

### Exemple 6 : Préparation d'un sel de méthionine

Un lait de chaux préparé à 20% de matière sèche, est mélangé en continu dans une conduite contenant un mélangeur statique avec une solution de méthionine à 20% de matière sèche.

Le temps de mise en contact est de 7 secondes. Le débit du lait de chaux est de 1,5Kg/H et celui de la solution de méthionine est de 6,0Kg/H.

Le mélange réactionnel est alors pulvérisé à l'aide d'une buse selon la connaissance de l'homme de l'art dans une tour d'atomisation simple effet avec une température d'entrée de 160°C et une température de sortie de 75°C.

Le produit obtenu a une teneur en HMTBA de 86,9%, en Ca²⁺ de 11,7%, et une humidité de 1,3%.

La granulométrie moyenne est de 35 µm et la densité de 300 g/L.

### Exemple 7 : Préparation d'un sel (HMTBA)₂Ca selon un procédé n'appartenant pas à la présente invention, et comparaison du produit obtenu avec un produit selon l'invention

Un sel d'HMTBA est préparé par addition, dans un récipient, de 100 g d'HMTBA à 88% de matière sèche, et 88 g de lait de chaux contenant 25 % de matière sèche.

Le mélange est réalisé sous agitation avec un mélangeur à hélice pendant 20 secondes.

Le mélange obtenu est laissé cristalliser pendant 20H à température ambiante puis séché en étuve à 105°C pendant 24H.

Le produit « R » ainsi obtenu est broyé à l'aide d'un mortier.

Les conditions d'obtention de l'échantillon « R » permettent d'obtenir un sel d'HMTBA cristallisé.

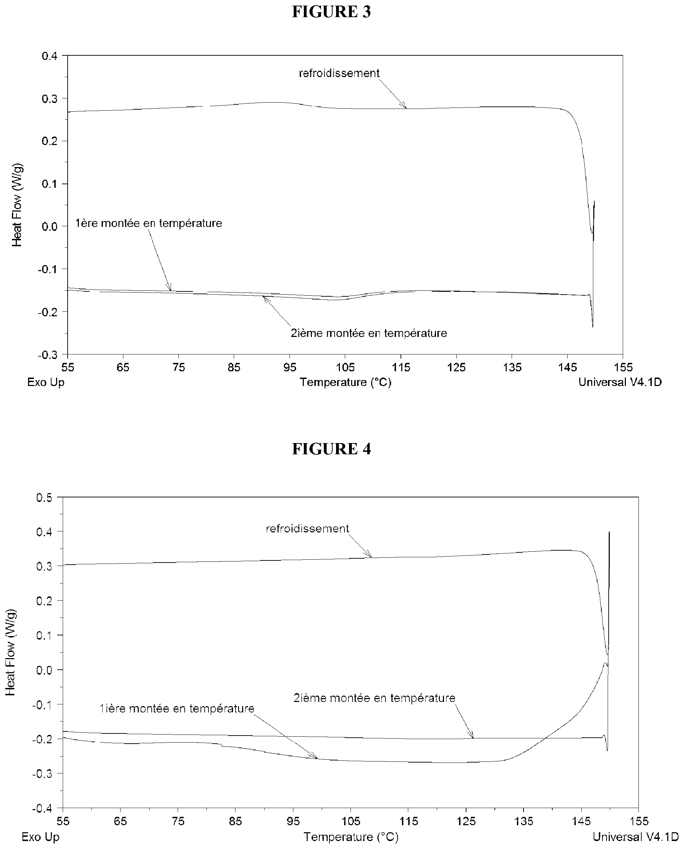

Ce produit « R » ainsi qu'un sel d'HMTBA obtenu selon les conditions de réalisation de l'exemple 2 appelé « T7 » sont analysés par diffraction aux rayons X et par calorimétrie différentielle à balayage en mode isotherme (en DSC - differential scanning calorimetry) La figure 3 obtenue par DSC montre que l'échantillon R présente un pic endothermique de faible énergie lors de la montée en température ainsi qu'un pic exothermique lors de la descente en température. Ces deux pics traduisent respectivement la fusion de cristaux puis la recristallisation de ces cristaux lors du refroidissement.

Un deuxième cycle de température donne les mêmes phénomènes endo et exo thermiques, permettant de vérifier qu'il s'agit bien de phénomène de fusion - cristallisation réversible. On ne voit pas d'autres phénomènes thermiques au cours de ces cycles de température. On en déduit que le composé « R » est un composés 100% cristallin.

Pour le composé issu de l'invention par DSC, la figure 4 montre qu'on observe un pic endothermique de très faible amplitude à 82,39°C qui pourrait correspondre à un phénomène de transition vitreuse (typique des systèmes amorphes) suivi de phénomènes endothermiques étendus non caractéristique de pics de fusion. On n'observe pas de phénomène thermique lors de la descente en température, donc il n'y a pas de recristallisation du produit à l'issu de la 1^{ère} montée en température.

On peut conclure que contrairement à l'échantillon « R » l'échantillon de l'invention est sous forme principalement amorphe.

Ces mêmes échantillons ont été analysés par diffraction aux rayons X.

Les figures 5b et 6b montrent que le premier pic est beaucoup plus large pour l'échantillon T7 que pour l'échantillon « R ». Ceci indique qu'il y a un ordre à courte distance mais pas à longue distance comme le suggère l'absence de pics suivants (notamment ceux à 5° et 9.2°). Ceci va dans le sens d'un échantillon totalement amorphe, avec seulement un ordre à courte distance. Le taux de cristallinité serait alors proche 0%.

### Exemple 8 : Autre préparation du sel (HMTBA)₂Ca

Un lait de chaux préparé à 26.2 % de matière sèche, est mélangé en continu par un dispositif de mélange par ultrasons à 126 kg/H avec une solution d'HMTBA à 88% de matière sèche à 143 Kg / H pour donner un mélange réactionnel de 59 % de matière sèche.

Le temps de mise en contact est de 20 secondes.

Le mélange réactionnel est pulvérisé à l'aide d'une buse selon la connaissance de l'homme de l'art, dans une tour d'atomisation simple effet avec une température d'entrée de 200°C et une température de sortie de 136°C

Le produit obtenu contient 83,5% d'HMTBA, 12,3% de Ca²⁺ et 2,7% d'eau.

### Exemple 9 : Autre préparation du sel (HMTBA)₂Ca

Un lait de chaux préparé à 45% de matière sèche, est mélangé en continu par une conduite contenant un mélangeur statique avec une solution d'HMTBA à 88% de matière sèche dans le ratio 20% de chaux (calculé sur la matière sèche engagée) et 80% d'HMTBA (calculé sur la matière sèche engagée).

Le temps de mise en contact est de 7 secondes.

Le mélange réactionnel est alors pulvérisé à l'aide d'une buse selon la connaissance de l'homme de l'art, dans une tour d'atomisation simple effet avec une température d'entrée de 160°C et une température de sortie de 90°C.

Le produit obtenu a une teneur en HMTBA de 85,3 %, en Ca²⁺ de 11,1%, et une humidité de 1,7%. La granulométrie moyenne est de 40 µm et la densité de 380 g/L.

### Exemple 10: Préparation d'un sel de sodium de l'acide aspartique (comparatif)

Une solution d'hydroxyde de sodium à 50 % en concentration massique est mélangée en continu dans une conduite contenant un mélangeur statique à 1,4 kg/h avec une suspension d'acide aspartique à 20 % en concentration massique à 11,6 kg/h.

Le temps de mise en contact est 15 secondes. Le produit est alors atomisé dans une tour d'atomisation simple effet avec une température d'entrée de 180 °C et une température de sortie de 95 °C.

Le produit obtenu est une poudre blanche, stable, qui présente un écoulement fluide et une bonne solubilité dans l'eau. La granulométrie moyenne est de 55 µm, l'humidité de 2,3 % et le pH en solution de 6,5.

### Exemple 11: Préparation d'un sel de calcium de l'acide aspartique

Un lait de chaux préparé à 30 % de matière sèche est mélangé en continu dans une conduite contenant un mélangeur statique à 1,9 kg/h avec une suspension d'acide aspartique à 20 % en concentration massique à 10,4 kg/h.

Le temps de mise en contact est 15 secondes. Le produit est alors atomisé dans une tour d'atomisation simple effet avec une température d'entrée de 180 °C et une température de sortie de 90 °C.

Le produit obtenu est une poudre blanche, stable, qui présente un écoulement fluide et une bonne solubilité dans l'eau. La granulométrie moyenne est de 35 µm, l'humidité de 2,9 % et le pH en solution de 6,8.

### Exemple 12: Préparation d'un sel de (HMTBA) de type 4

Un lait de chaux préparé à 30 % de matière sèche est mélangé en continu dans une conduite contenant un mélangeur statique à 70 kg/h avec une solution d'HMTBA à 88 % de matière sèche à 193 kg/h.

Le temps de mise en contact est de 15 secondes. Le produit est alors atomisé dans une tour d'atomisation multiple effet avec une température d'entrée de 180 °C, une température de sortie de 95 °C, et un recyclage des particules fines en zone de pulvérisation.

Le produit obtenu a une teneur de 91,6 % en HMTBA, de 5,7 % en calcium, et une humidité de 1,5 %.

### Exemple 13 : Préparation d'un sel de Cu de HMTBA (comparatif)

Une suspension d'hydroxyde de cuivre à 35% de matière sèche est mélangée en continu dans une conduite contenant un mélangeur statique à 2,8 kg/H, avec une solution d'HMTBA à 88% de matière sèche à 3,5 Kg / H.

Le temps de mise en contact lors de l'atomisation réactive est de 8 secondes.

Le mélange réactionnel est alors pulvérisé à l'aide d'une buse selon la connaissance de l'homme de l'art dans une tour d'atomisation simple effet avec une température d'entrée de 140°C et une température de sortie de 80°C.

Le produit obtenu a une teneur en HMTBA de 81,8%, une teneur en Cu²⁺ de 15,4% et une humidité de 1,2%.

La granulométrie moyenne est de 40 µm et la densité de 420 g/L.

### Exemple 14 : Utilisation d'un sel (HMTBA)₂Ca selon l'invention pour l'alimentation de poules pondeuses

### Résumé

Des poules pondeuses ont été nourries soit avec de la DL-méthionine (DLM), soit avec un sel HMTBA-Ca selon l'invention, soit avec une combinaison (50/50) des deux pendant 6 semaines. Les performances de ponte et les paramètres relatifs aux oeufs ont été mesurés pendant les 6 semaines. HMTBA-Ca est aussi efficace que DLM pour la plupart des paramètres, mais améliore l'efficacité de ponte (l'indice de consommation ou le poids moyen des oeufs). HMTBA-Ca contribue à obtenir une masse d'albumine plus importante que celle obtenue avec DLM

La combinaison de HMTBA-Ca et de DLM donne des résultats intermédiaires sur les paramètres de performance ou les paramètres relatifs aux oeufs.

### Conditions expérimentales

Quatre-vingts poules pondeuses de 45 semaines ont été logées pendant 6 semaines et ont été réparties au hasard en trois groupes égaux (20 par groupe). Chaque poule était logée dans une cage individuelle sous température (20 ± 2 °C) et conditions d'éclairage contrôlées. Toutes les poules se sont vues proposer un régime basal analogue, avec ou sans l'ajout de DLM, de HMTBA-Ca ou d'un mélange 50/50 (HMTBA-Ca: DLM) (tableaux 1 et 2) pour donner un supplément en équivalent méthionine de 0,13% pour toutes les compositions alimentaires. Toutes les poules pondeuses avaient libre accès à l'eau potable et ont été nourries tout au long de la période de 6 semaines d'expérimentation. La performance de ponte d'oeufs, y compris les composants des oeufs, a été mesurée au cours de la période de 6 semaines.

**Tableau 1 : Composition du régime alimentaire (%)**

| | Groupe 1 | Groupe 2 | Groupe 3 |
|---|---|---|---|
| Matière première | % | % | % |
| Maïs | 50 | 50 | 50 |
| Blé | 16.1 | 16.1 | 16.1 |
| Tourteau de soja 48 | 22.07 | 22.07 | 22.07 |
| Huile de soja | 0.95 | 0.95 | 0.95 |
| DL-Methionine (NP99) | 0.13 | 0 | 0.07 |
| HMTBA-Ca | 0 | 0.15 | 0.08 |
| Calcaire | 8.12 | 8.12 | 8.12 |
| Phosphate bicalcique | 1.48 | 1.48 | 1.48 |
| Sel | 0.35 | 0.35 | 0.35 |
| Prémix | 0.8 | 0.8 | 0.8 |
| *Total* | *100* | *100* | *100* |
| | | | |

| Nutriments | | | |
|---|---|---|---|
| Protéines brutes (%) | 16.5 | 16.5 | 16.5 |
| Energie métabolisable(Kcal/kg) | 2730 | 2730 | 2730 |
| Calcium (%) | 3.508 | 3.508 | 3.508 |
| Phosphore disponible (%) | 0.331 | 0.331 | 0.331 |
| Dig. Méthionine (%) | 0.37 | 0.37 | 0.37 |
| Dig. M+C (%) | 0.603 | 0.603 | 0.603 |
| Dig.Lysine (%) | 0.722 | 0.722 | 0.722 |

**Tableau 2 : Niveaux de supplémentation en méthionine : attendus et mesurés (%)**

| | Groupe 1 | Groupe 2 | Groupe 3 |
|---|---|---|---|
| Méthionine totale (%) | 0.35 | 0.24 | 0.28 |
| Méthionine ajoutée (%) | 0.11 | 0.00 | 0.06 |
| HMTBA ajouté (%) | 0.00 | 0.11 | 0.07 |
| *Total en équivalent Méthionine(%)* | *0.35* | *0.35* | *0.35* |

### Performance de ponte et résultats relatifs aux caractéristiques des oeufs

Les données relatives à la performance de ponte et à la composition des oeufs (albumen et jaune) sont présentées dans le tableau 3 ci-dessous.

Les poules pondeuses nourries au sel HMTBA-Ca produisent une masse quotidienne d'oeufs plus importante que celles nourries avec DLM, car la masse moyenne des oeufs avec HMTBA-Ca est supérieure de 2,3% par rapport à celle obtenue avec DLM. La proportion d'albumen et de jaune n'est pas significativement affectée par la source de méthionine, mais les oeufs des poules nourries avec HMTBA-Ca contiennent plus d'albumen (+3%) par rapport aux oeufs des poules nourries à la DLM.

**Tableau 3: Résultats relatifs aux différentes supplémentations en méthionine.**

| | Groupe 1 | | Groupe 2 | | Groupe 3 | | ANOV A |
|---|---|---|---|---|---|---|---|
| | DLM | | HMTBA-Ca | | Mélange 50/50 | | |
| | Moyenne | Erreur standard | Moyenne | Erreur standard | Moyenne | Erreur standard | valeur de p |
| Poids corporel initial (g/poule) | 1724.7 | *126.7* | 1738.6 | *135.6* | 1729.3 | *110.5* | *0.94* |
| Prise alimentaire quotidienne (g/jour) | 112.4 | *8.1* | 112.7 | 5.9 | 113.0 | 5.9 | *0.97* |
| Taux de ponte (%) | 95% | *5*% | 95% | *4%* | 96% | *4%* | *0.63* |
| Poids moyen des oeufs(g/oeuf) | 63.4 | *4.0* | 64.9 | *3.6* | 64.1 | *3.1* | *0.47* |
| Masse d'oeufs (g/jour) | 60.3 | *4.2* | 62.0 | 4.6 | 61.8 | *4.0* | *0.41* |
| Albumen (g/oeuf) | 38.38 | 2.78 | 39.53 | *2.67* | 39.14 | 2.69 | *0.71* |
| Jaune (g/oeuf) | 16.38 | 1.08 | 16.47 | *0.95* | 16.18 | *1.07* | *0.74* |
| Indice de consommation( g alim/g oeuf) | 1.87 | *0.12* | 1.82 | *0.12* | 1.83 | *0.10* | *0.44* |
| Poids corporel final (g/poule) | 1834.9 | *98.0* | 1897.2 | *150.0* | 1850.2 | *112.8* | *0.28* |

### Exemple 15: Utilisation d'un sel (HMTBA)₂Ca selon l'invention pour l'alimentation de poulets en croissance

### Résumé

Des poulets en croissance ont été supplémentés (0,3%) soit en DLM, soit en HMTBA soit en sel HMTBA-Ca selon l'invention, pendant 46 jours. Le HMTBA et plus particulièrement le HMTBA-Ca ont présenté les meilleures performances de croissance. En outre, le HMTBA-Ca a montré une digestibilité des nutriments nettement meilleure que celle obtenue avec les deux autres formes de méthionine.

### Conditions expérimentales

Cent quarante-sept poulets commerciaux (1 jour d'âge, 48 g) ont été logés du jour 1 au jour 46, incluant la phase de démarrage (jours 1-21) et la phase de finition (jours 22-46). Tous les poulets ont été aléatoirement répartis en sept groupes égaux (21 par groupe), et chaque groupe se composait de trois sous-groupes de 7 oiseaux chacun. Chaque sous-groupe a été logé dans un parquet à la température (28 ± 2°C) et les conditions d'éclairage contrôlées. Tous les poulets se sont vus proposer un régime basal analogue, avec ou sans l'ajout de DLM, HMTBA ou HMTBA-Ca à des niveaux de 0 (témoin) ou 0,3 % en phase de démarrage et 0 ou 0,24% en phase de finition (tableau 4). Tous les poulets ont eu libre accès à la nourriture et à l'eau potable tout au long de la période d'essai de 46 jours. La masse des poulets a été mesurée toutes les semaines et la prise alimentaire a été suivie tout au long de l'expérience.

Les excréments des poulets ont été recueillis pendant les 3 jours suivants le 42^{ème} jour d'expérience ont été congelés et stockées (à -20 °C) pour analyses chimiques ultérieures.

A la fin de l'expérience, les oiseaux ont été pesés individuellement et sacrifiés. La graisse abdominale, les muscles de la cuisse et du filet ont été prélevés, lyophilisés et pesés.

**Tableau 4 : Composition de l'alimentation**

| Composition (%) | Phase de démarrage (jours 0-21) | Phase de finition (jours 21-46) |
|---|---|---|
| Maïs | 50.42 | 49.72 |
| Tourteau de soja | 37.00 | 32.00 |
| Blé | 4.00 | 8.00 |
| NaCl | 0.34 | 0.34 |
| Phosphate bicalcique | 1.90 | 1.50 |
| Calcaire | 1.00 | 1.10 |
| Choline | 0.04 | 0.04 |
| Huile | 5.00 | 7.00 |
| Sources de méthionine¹ | 0 - 0.30 | 0 - 0.24 |
| Prémix vitamines-minéraux ² | 0.30 | 0.30 |
| Composition en nutriments | | |
| ME, Mcal/kg | 3.04 | 3.18 |
| CP, % | 20.97 | 19.27 |
| Lys, % | 1.12 | 1.10 |
| Mét + cystéine, % | 0.67 | 0.63 |
| Mét | 0.33 | 0.31 |
| P disponible, % | 0.44 | 0.37 |
| Ca, % | 0.97 | 19.27 |
| P total, % | 0.69 | 0.61 |

| | | |
|---|---|---|
| ¹HMTBA, DLM ou HMTBA-Ca ² par kg d'alimentation : vitamine A (acétate de rétinol), 1.500 UI; cholécalciférol, 200UI ; vitamine E (DL-α-tocophérol), 10 UI; riboflavine, 3,5 mg, acide pantothénique, 10 mg; niacine, 30 mg; cobalamine, 10 g, chlorure de choline, 1.000 mg; biotine, 0,15 mg, acide folique, 0,5 mg, thiamine, 1,5 mg; pyridoxine, 3,0 mg, Fe, 80 mg; Zn, 40 mg, Mn,60 mg, I , 0,18 mg; Cu, 8 mg; Se, 0,15 mg. | | |

### Résultats

### Performances relatives à la croissance et composition des carcasses

Les données relatives aux performances de croissance et à la composition des carcasses sont résumées dans les tableaux 5 et 6. Le poids corporel final et le gain de poids corporel sont significativement plus élevés chez les poulets nourris avec HMTBA 0,3% par rapport au groupe témoin. Il n'y a pas de différence significative dans l'apport alimentaire au niveau des sept groupes, mais un indice de consommation plus bas a été observé dans le groupe HMTBA 0,3%. Au cours de l'expérience, le poids des muscles de la cuisse et du filet des poulets nourris avec HMTBA ou HMTBA-Ca étaient significativement plus élevés que ceux nourris avec la DLM. Aucune différence significative n'a été observée au niveau du poids de la graisse abdominale en fonction de la source de méthionine, à l'exception d'une augmentation du pourcentage de graisse abdominale chez le groupe supplémenté en HMTBA-Ca (0,3%).

**Tableau 5: Résultats relatifs à la supplémentation en DLM, HMTBA ou HMTBA-Ca sur des poulets de 46 jours*.**

| Groupe | Poids corporel final (kg/poulet) | Gain en poids corporel (g/poulet/jour) | Prise alimentaire (g/poulet/jour) | Alimentation: gain (g:g) |
|---|---|---|---|---|
| Contrôle | 3.05±0.10^{a} | 67.85±2.26^{a} | 134.85±3.72 | 1.99±0.01 |
| 0.3%DLM | 3.17±0.15^{ab} | 70.47±3.37^{ab} | 141.51±0.24 | 2.01±0.10 |
| 0.3%HMTBA | 3.44±0.02^{b} | 76.41±0.40^{b} | 142.23±8.46 | 1.86±0.12 |
| 0.3%HMTBA-Ca | 3.38±0.28^{ab} | 75.08±6.18^{ab} | 144.53±20.93 | 1.91±0.12 |

| | | | | |
|---|---|---|---|---|
| * Les valeurs sont exprimées en moyenne ± écart-type. Chaque groupe représente 21 poulets à l'âge de 46 jours. ^{a,b,c}Au sein d'une même colonne, les valeurs avec des exposants différents sont significativement différentes (P <0,05). | | | | |

**Tableau 6 : Effet de DLM, HMTBA ou HMTBA-Ca sur la composition de la carcasse des poulets de 46 jours*.**

| Groupe | DL (g) | PDL (%) | DB (g) | PDB (%) | PDAT (%) |
|---|---|---|---|---|---|
| Contrôle | 549.42±15.32^{a} | 20.31±0.16^{ab} | 521.69±64.00^{a} | 20.88±1.63^{ab} | 1.77±0.62^{a} |
| 0.3%DLM | 586.03±23.43^{ab} | 20.12±0.22^{a} | 596.88±56.68^{abc} | 22.77±1.44^{b} | 1.88±0.43^{a} |
| 0.3%HMTBA | 648.98±27.92^{c} | 21.98±0.97^{c} | 669.66±50.16^{c} | 22.77±1.44^{b} | 2.07±0.63^{ab} |
| 0.3%HMTBA-Ca | 588.64±38.07^{ab} | 22.06±1.64^{c} | 617.54±45.21^{bc} | 22.98±3.00^{b} | 2.49±0.59^{b} |

| | | | | | |
|---|---|---|---|---|---|
| DL, poids des muscles de la cuisse DB, poids des muscles du filet PDL, rapport du poids des muscles de la cuisse (sans os ni peau) sur celui du poulet éviscéré PDB, rapport du poids des muscles du filet (sans os ni peau) sur celui du poulet éviscéré PDAT, rapport du poids de la graisse abdominale sur celui du poulet éviscéré * Les valeurs sont exprimées en moyenne ± écart-type. Chaque groupe représente 21 poulets à l'âge de 46 jours. ^{a,b,c}Au sein d'une même colonne, les valeurs avec des exposants différents sont significativement différentes (P <0,05). | | | | | |

### Digestibilité apparente

Comme le montre le tableau 7, les régimes supplémentés en équivalent de méthionine ont entraîné une augmentation significative de la digestibilité apparente de la matière sèche, des protéines brutes et matières grasses brutes dans les poulets, et dans l'ordre HMTBA-Ca> HMTBA> DLM. En revanche, la digestibilité apparente de cendres était significativement plus faible que pour les groupes témoins. Les régimes alimentaires contenant HMTBA-Ca ou HMTBA ont permis d'améliorer la digestibilité apparente par rapport à des régimes contenant DLM, ceci étant principalement dû à l'activité dans l'ensemble plus élevée des enzymes digestives dans le duodénum et le jéjunum.

**Tableau 7 : digestibilité apparente (%) de la matière sèche, des protéines brutes, des matières grasses brutes et des cendres, dans des poulets de 46 jours supplémentés en DLM, HMTBA ou HMTBA-Ca*.**

| Groupe | matière sèche | protéines brutes | matières grasses brutes | Cendres |
|---|---|---|---|---|
| Contrôle | 68.39±1.31^{a} | 44.23±2.95^{a} | 81.93±1.90^{a} | 86.03±3.98^{d} |
| 0.3%DLM | 78.22±1.70^{c} | 63.70±2.85^{cd} | 86.15±0.93 | 56.02±1.01^{b} |
| 0.3%HMTBA | 79.02±1.61^{cd} | 62.40±2.89^{cd} | 87.80±1.39^{bc} | 55.83±0.16^{b} |
| 0.3%HMTBA-Ca | 81.80±0.32^{d} | 66.44±0.37^{cd} | 90.28±0.20^{c} | 44.62±0.89^{a} |

| | | | | |
|---|---|---|---|---|
| * Les valeurs sont exprimées en moyenne ± écart-type pour 10 poulets. ^{a,b,c}Au sein d'une même colonne, les valeurs avec des exposants différents sontsignificativement différentes (P <0,05). | | | | |

### Conclusion

La présente étude montre que les régimes alimentaires comprenant HMTBA-Ca ou HMTBA conduisent à l'amélioration de la croissance et de la composition de la carcasse par rapport à des régimes contenant de la DLM. Le régime supplémenté en HMTBA-Ca (0,3%) a conduit au poids le plus élevé en muscles de la cuisse et du filet, par rapport aux autres groupes. En outre, le régime supplémenté en HMTBA-Ca a conduit à une plus grande digestibilité apparente par rapport au groupe supplémenté en DLM. Les résultats montrent que la supplémentation en HMTBA ou HMTBA-Ca a entraîné une augmentation du gain de poids corporel, du poids des muscles de la cuisse et du filet en l'absence de toute réduction de la prise alimentaire par l'induction de l'activité des enzymes digestives et par la régulation de l'absorption intestinale des nutriments essentiels.

## Revendications

1. Particule, sous la forme d'une sphère homogène ou d'une fraction de sphère homogène, constituée essentiellement d'un complexe, notamment un sel, entre un acide ou un anion correspondant et au moins un métal ou un cation métallique correspondant,
ledit acide ou anion correspondant étant l'acide 2-hydroxy-4-méthyl-thio-butanoïque (HMTBA) ou le 2-hydroxy-4-méthyl-thio-butanoate,
ledit métal ou cation métallique étant divalent, choisi parmi le Ca ou le Ca²⁺,
ladite particule présentant une fraction amorphe dont la masse représente au moins 50%, en particulier au moins 70%, plus particulièrement au moins 90%, de la masse totale de ladite particule,
ladite particule étant substantiellement dépourvue d'acide ou d'anion non complexé et de métal ou de cation métallique non complexé,
le rapport de la masse dudit acide ou anion non complexé et/ou dudit au moins un métal ou cation non complexé sur la masse totale de ladite particule étant inférieure à 20 %, et variant en particulier de 0 à 5 %, plus particulièrement de 0 à 1 %.

2. Particule selon la revendication 1, dans laquelle ledit complexe est de formule la suivante :
(Acide)ₙM (Ia)
où
M représente ledit métal,
n étant égal à 2 lorsque ledit métal est divalent,
ledit complexe étant en particulier un sel, plus particulièrement un sel de formule (HMTBA)₂Ca.

3. Composition pulvérulente de particules, lesdites particules étant telles que définies dans l'une quelconque des revendications 1 à 2,
la taille granulométrique desdites particules variant de 10 à 3000 µm, en particulier de 20 à 300 µm, en moyenne granulométrique [Dv(0,5)], et/ou
ladite composition possédant un indice d'écoulement variant de 4 à 18 [indice flodex], et/ou
ladite composition étant telle que l'essentiel des plus fines particules de la composition soit aggloméré, collé ou fusionné à d'autres particules de la composition, de façon à ce qu'il ne reste pas plus de 10 % de fines particules de la composition, inférieures à 100 µm,.

4. Procédé de préparation d'une composition pulvérulente de particules selon l'une quelconque des revendications 1 à 3, constituées essentiellement d'un complexe, notamment un sel, entre un acide ou un anion correspondant et au moins un métal ou un cation métallique correspondant, ledit procédé comprenant une étape de mise en contact dudit acide avec une source minérale dudit métal ou cation correspondant dans une tour de séchage par atomisation pour obtenir la formation dudit complexe, notamment dudit sel, et initier sa précipitation,
ledit acide ou anion correspondant étant l'acide 2-hydroxy-4-méthyl-thio-butanoïque (HMTBA), ou le 2-hydroxy-4-méthyl-thio-butanoate,
ledit métal ou cation métallique étant divalent, choisi parmi le Ca ou le Ca²⁺,
ladite particule présentant une fraction amorphe dont la masse représente au moins 50%, en particulier au moins 70%, plus particulièrement au moins 90%, de la masse totale de ladite particule.

5. Procédé selon la revendication 4, comprenant une étape de pulvérisation au cours de laquelle la précipitation dudit complexe, notamment dudit sel, se poursuit,
les étapes de mise en contact et de pulvérisation étant notamment réalisées à l'aide du même dispositif, en particulier un dispositif comprenant un organe de pulvérisation tournant, la mise en contact étant immédiatement suivie de la pulvérisation.

6. Procédé selon l'une quelconque des revendications 4 à 5, comprenant une étape de séchage par atomisation dudit complexe, notamment dudit sel, au cours de laquelle la précipitation dudit complexe, notamment dudit sel, se poursuit jusqu'à solidification complète de la particule.

7. Procédé selon l'une quelconque des revendications 4 à 6, comprenant les étapes suivantes :
- mise en contact dudit acide avec une source minérale dudit métal ou cation correspondant dans une tour de séchage par atomisation pour obtenir la formation dudit complexe, notamment dudit sel, et initier sa précipitation ;
- pulvérisation dudit complexe, notamment dudit sel, en cours de précipitation, au cours de laquelle ladite précipitation se poursuit, pour obtenir un ensemble de particules pulvérisées ;
- séchage par atomisation dudit ensemble de particules pulvérisées, au cours de laquelle ladite précipitation se poursuit jusqu'à solidification complète de la particule, pour obtenir une composition pulvérulente stable ;
- récupération de ladite composition pulvérulente ;
ledit procédé comprenant notamment les étapes suivantes :
- mise en contact dudit acide avec une source minérale dudit métal ou cation correspondant dans une tour de séchage par atomisation, à l'aide d'un dispositif permettant ladite mise en contact, pour obtenir la formation dudit complexe, notamment dudit sel, et initier sa précipitation ;
- pulvérisation dudit complexe, notamment dudit sel, en cours de précipitation, au cours de laquelle ladite précipitation se poursuit, à l'aide d'un dispositif de pulvérisation, pour obtenir un ensemble de particules pulvérisées, ladite pulvérisation étant immédiatement subséquente à la mise en contact, ledit dispositif de pulvérisation étant identique à celui permettant la mise en contact, en particulier un dispositif comprenant un organe de pulvérisation tournant;
- séchage par atomisation dudit ensemble de particules pulvérisées, au cours de laquelle ladite précipitation se poursuit jusqu'à solidification complète de la particule, pour obtenir une composition pulvérulente, stable ;
- récupération de ladite composition pulvérulente.

8. Procédé selon l'une quelconque des revendications 4 à 7, dans lequel :
- ladite mise en contact se fait par mélange d'un milieu aqueux, en particulier une solution aqueuse, contenant ledit acide et d'un milieu aqueux, en particulier une solution ou une suspension aqueuse contenant ledit métal ou cation, et/ou
- la mise en contact dudit acide avec une source minérale dudit métal ou cation correspondant est réalisée en continu, ladite mise en contact en continu étant en particulier réalisée dans un dispositif tel que les mélangeurs statiques ou dynamiques, en particulier les malaxeurs, les extrudeurs, et les mélangeurs sans pièce interne, comme les mélangeurs à ultrason.

9. Procédé selon l'une quelconque des revendications 4 à 8, dans lequel ledit complexe est de formule la suivante :
(Acide)ₙM (Ia)
où M représente ledit métal,
ledit complexe étant en particulier un sel, plus particulièrement un sel de formule (HMTBA)₂Ca,.

10. Procédé selon l'une quelconque des revendications 4 à 9, dans lequel lesdites particules sont constituées essentiellement d'un sel entre un anion d'un acide et au moins un cation métallique, en particulier :
- ledit anion étant le 2-hydroxy-4-méthyl-thio-butanoate et ledit cation étant Ca²⁺, la source de Ca²⁺ étant choisie parmi la chaux, le lait de chaux, la chaux éteinte, l'hydrogénocarbonate de calcium et le carbonate de calcium, ou étant Ca(OH)₂.

11. Procédé selon l'une quelconque des revendications 4 à 10, comprenant une étape supplémentaire d'agglomération, ou dans lequel ladite tour de séchage par atomisation est une tour de séchage par atomisation multiple effet, ladite étape de mise en réaction dudit acide avec ladite source métallique minérale comprenant également une agglomération.

## Patentansprüche

1. Partikel, in der Form einer homogenen Sphäre oder einer Fraktion einer homogenen Sphäre, im Wesentlichen bestehend aus einem Komplex, insbesondere einem Salz, zwischen einer Säure oder einem entsprechenden Anion und mindestens einem Metall oder einem entsprechenden metallischen Kation,
wobei die Säure oder das entsprechende Anion 2-Hydroxy-4-methylthiobutansäure (HMTBA) oder 2-Hydroxy-4-methylthiobutanoat ist,
wobei das Metall oder metallische Kation zweiwertig ist, ausgewählt aus Ca oder Ca²⁺,
wobei das Partikel eine amorphe Fraktion aufweist, deren Masse mindestens 50 %, insbesondere mindestens 70 %, bevorzugter mindestens 90 %, der Gesamtmasse des Partikels beträgt,
wobei das Partikel im Wesentlichen keine nichtkomplexierte Säure oder kein nicht-komplexiertes Anion und kein nicht-komplexiertes Metall oder kein nicht-komplexiertes metallisches Kation umfasst,
wobei das Masseverhältnis der nicht-komplexierten Säure oder des nicht-komplexierten Anions und/oder des mindestens einen nicht-komplexierten Metalls oder des nicht-komplexierten Kations, bezogen auf die Gesamtmasse des Partikels, weniger als 20 % beträgt, und insbesondere von 0 bis 5 %, bevorzugter von 0 bis 1 %, variiert.

2. Partikel nach Anspruch 1, wobei der Komplex die folgende Formel aufweist:
(Säure)ₙM (Ia),
wobei
M das Metall darstellt,
n gleich 2 ist, wenn das Metall zweiwertig ist,
wobei der Komplex insbesondere ein Salz, bevorzugter ein Salz mit der Formel (HMTBA)₂Ca, ist.

3. Pulverzusammensetzung aus Partikeln, wobei die Partikel wie in einem der Ansprüche 1 bis 2 definiert sind,
wobei die granulometrische Größe der Partikel von 10 bis 3000 µm, insbesondere von 20 bis 300 µm, im granulometrischen Mittel [Dv(0,5)] variiert, und/oder wobei die Zusammensetzung einen Flussindex aufweist, der von 4 bis 18 variiert [Flodex-Index], und/oder wobei die Zusammensetzung derart ist, dass das Wesentliche der feineren Partikel der Zusammensetzung mit anderen Partikeln der Zusammensetzung derart agglomeriert, verklebt oder verschmolzen ist, dass nicht mehr als 10 % der feinen Partikel der Zusammensetzung, die kleiner sind als 100 µm, zurückbleiben.

4. Verfahren zur Herstellung einer Pulverzusammensetzung aus Partikeln gemäß einem der Ansprüche 1 bis 3, im Wesentlichen bestehend aus einem Komplex, insbesondere einem Salz, zwischen einer Säure oder einem entsprechenden Anion und mindestens einem Metall oder einem entsprechenden metallischen Kation, wobei das Verfahren einen Schritt des Inkontaktbringens der Säure mit einer mineralischen Quelle des Metalls oder entsprechenden Kations in einem Trockenturm durch Zerstäubung umfasst, um die Bildung des Komplexes, insbesondere des Salzes, zu erhalten und seine Fällung zu initiieren,
wobei die Säure oder das entsprechende Anion 2-Hydroxy-4-methylthiobutansäure (HMTBA) oder 2-Hydroxy-4-methylthiobutanoat ist,
wobei das Metall oder metallische Kation zweiwertig ist, ausgewählt aus Ca oder Ca²⁺,
wobei das Partikel eine amorphe Fraktion aufweist, deren Masse mindestens 50 %, insbesondere mindestens 70 %, bevorzugter mindestens 90 %, der Gesamtmasse des Partikels beträgt.

5. Verfahren nach Anspruch 4, umfassend einen Schritt des Pulverisierens, in dem die Fällung des Komplexes, insbesondere des Salzes, erfolgt,
wobei die Schritte des Inkontaktbringens und des Pulverisierens insbesondere mit Hilfe derselben Vorrichtung durchgeführt werden, insbesondere einer Vorrichtung, die ein sich drehendes Pulverisierungselement umfasst, wobei das Inkontaktbringen unmittelbar von dem Pulverisieren gefolgt wird.

6. Verfahren nach einem der Ansprüche 4 bis 5, umfassend einen Schritt des Trocknens des Komplexes, insbesondere des Salzes, durch Zerstäubung, in dem die Fällung des Komplexes, insbesondere des Salzes, bis zur vollständigen Verfestigung des Partikels erfolgt.

7. Verfahren nach einem der Ansprüche 4 bis 6, umfassend die folgenden Schritte:
- Inkontaktbringen der Säure mit einer mineralischen Quelle des Metalls oder entsprechenden Kations in einem Trockenturm durch Zerstäubung, um die Bildung des Komplexes, insbesondere des Salzes, zu erhalten und seine Fällung zu initiieren;
- Pulverisieren des Komplexes, insbesondere des Salzes, während der Fällung, wobei die Fällung währenddessen erfolgt, um eine Einheit pulverisierter Partikel zu erhalten;
- Trocknen der Einheit der pulverisierten Partikel durch Zerstäubung, wobei die Fällung währenddessen bis zur vollständigen Verfestigung des Partikels erfolgt, um eine stabile Pulverzusammensetzung zu erhalten;
- Gewinnen der Pulverzusammensetzung;
wobei das Verfahren insbesondere die folgenden Schritte umfasst:
- Inkontaktbringen der Säure mit einer mineralischen Quelle des Metalls oder entsprechenden Kations in einem Trockenturm durch Zerstäubung, mit Hilfe einer Vorrichtung, die das Inkontaktbringen gestattet, um die Bildung des Komplexes, insbesondere des Salzes, zu erhalten und seine Fällung zu initiieren;
- Pulverisieren des Komplexes, insbesondere des Salzes, während der Fällung, wobei die Fällung währenddessen erfolgt, mit Hilfe einer Pulverisierungsvorrichtung, um eine Einheit pulverisierter Partikel zu erhalten, wobei die Pulversierung unmittelbar auf das Inkontaktbringen folgt, wobei die Pulverisierungsvorrichtung mit jener identisch ist, die das Inkontaktbringen gestattet, insbesondere einer Vorrichtung, die ein sich drehendes Pulverisierungselement umfasst;
- Trocknen der Einheit der pulverisierten Partikel durch Zerstäubung, wobei die Fällung währenddessen bis zur vollständigen Verfestigung des Partikels erfolgt, um eine stabile Pulverzusammensetzung zu erhalten;
- Gewinnen der Pulverzusammensetzung.

8. Verfahren nach einem der Ansprüche 4 bis 7, wobei:
- das Inkontaktbringen durch Mischen eines wässrigen Mediums, insbesondere einer wässrigen Lösung, welche die Säure enthält, und eines wässrigen Mediums, insbesondere einer wässrigen Lösung oder Suspension, die das Metall oder Kation enthält, erfolgt, und/oder
- das Inkontaktbringen der Säure mit einer mineralischen Quelle des Metalls oder entsprechenden Kations kontinuierlich durchgeführt wird, wobei das kontinuierliche Inkontaktbringen insbesondere in einer Vorrichtung durchgeführt wird, wie statischen oder dynamischen Mischern, insbesondere Knetern, Extrudern, und Mischern ohne Innenstück, wie Ultraschallmischern.

9. Verfahren nach einem der Ansprüche 4 bis 8, wobei der Komplex die folgende Formel aufweist:
(Säure)ₙM (Ia),
wobei M das Metall darstellt,
wobei der Komplex insbesondere ein Salz, bevorzugter ein Salz mit der Formel (HMTBA)₂Ca, ist.

10. Verfahren nach einem der Ansprüche 4 bis 9, wobei die Partikel im Wesentlichen aus einem Salz zwischen einem Anion einer Säure und mindestens einem metallischen Kation bestehen, wobei insbesondere:
- das Anion 2-Hydroxy-4-methylthiobutanoat ist, und das Kation Ca²⁺ ist, wobei die Quelle von Ca²⁺ ausgewählt wird aus Kalk, Kalkmilch, gelöschtem Kalk, Calciumhydrogencarbonat und Calciumcarbonat, oder Ca(OH)₂ ist.

11. Verfahren nach einem der Ansprüche 4 bis 10, umfassend einen ergänzenden Agglomerationsschritt, oder wobei der Trockenturm durch Zerstäubung ein Trockenturm durch Zerstäubung mit mehrfachem Effekt ist, wobei der Schritt des Umsetzens der Säure mit der mineralischen metallischen Quelle auch eine Agglomeration umfasst.

## Claims

1. Particle, in the form of a homogeneous sphere or a fraction of a homogeneous sphere, consisting essentially of a complex, in particular a salt, between an acid or a corresponding anion and at least one metal or a corresponding metal cation,
said acid or corresponding anion being 2-hydroxy-4-methyl-thiobutanoic acid (HMTBA) or 2-hydroxy-4-methyl-thiobutanoate,
said metal or metal cation being divalent, chosen from Ca or Ca²⁺,
said particle having an amorphous fraction the mass of which represents at least 50%, in particular at least 70%, more particularly at least 90%, of the total mass of said particle, said particle being substantially devoid of uncomplexed acid or anion and of uncomplexed metal or metal cation,
the ratio of the mass of said uncomplexed acid or anion and/or of said at least one uncomplexed metal or cation to the total mass of said particle being below 20%, and in particular being in the range from 0 to 5%, more particularly from 0 to 1%.

2. Particle according to claims 1, wherein said complex has the following formula (Ia):
(Acid)ₙM (Ia)
where
M represents said metal,
n being equal to 2 when said metal is divalent,
said complex being in particular a salt, more particularly a salt of formula (HMTBA)₂Ca.

3. Pulverulent composition of particles, said particles being as defined in any one of claims 1 to 2,
the granulometric size of said particles being in the range from 10 to 3000 µm, in particular from 20 to 300 µm, in granulometric average [Dv(0.5)], and/or
said composition having a flowability index that is in the range from 4 to 18 [Flodex index], and/or
said composition being such that most of the finest particles of the composition are either agglomerated to, glued to or fused to other particles in the composition, so that there remains no more than 10% of the fine particles of the composition which are smaller than 100 µm.

4. Method for preparing a pulverulent composition of particles according to any one of claims 1 to 3, consisting essentially of a complex, in particular a salt, between an acid or a corresponding anion and at least one metal or a corresponding metal cation, said method comprising a step of bringing said acid into contact with a mineral source of said metal or corresponding cation in a spray-drying tower in order to obtain the formation of said complex, in particular of said salt, and initiate its precipitation,
said acid or corresponding anion being 2-hydroxy-4-methyl-thiobutanoic acid (HMTBA), or 2-hydroxy-4-methyl-thiobutanoate,
said metal or metal cation being divalent, chosen from Ca or Ca²⁺,
said particle having an amorphous fraction the mass of which represents at least 50%, in particular at least 70%, more particularly at least 90%, of the total mass of said particle.

5. Method according to claim 4, comprising a spraying step, during which precipitation of said complex, in particular of said salt, takes place,
the contacting and spraying steps in particular being carried out using the same device, in particular a device comprising a rotating spraying element, the contacting step being followed immediately by the spraying step.

6. Method according to either of claims 4 and 5, comprising a step of spray-drying of said complex, in particular of said salt, during which precipitation of said complex, in particular of said salt, proceeds until there is complete solidification of the particle.

7. Method according to any one of claims 4 to 6, comprising the following steps:
- bringing said acid into contact with a mineral source of said metal or corresponding cation in a spray-drying tower in order to obtain the formation of said complex, in particular of said salt, and initiate its precipitation;
- spraying said complex, in particular said salt, undergoing precipitation, during which said precipitation continues to obtain a set of sprayed particles;
- spray-drying said set of sprayed particles, during which said precipitation continues until there is complete solidification of the particle, to obtain a stable pulverulent composition;
- recovery of said pulverulent composition;
said method in particular comprising the following steps:
- bringing said acid into contact with a mineral source of said metal or corresponding cation in a spray-drying tower, using a device that allows said contact, in order to obtain the formation of said complex, in particular of said salt, and initiate its precipitation;
- spraying said complex, in particular said salt, undergoing precipitation, during which said precipitation takes place, using a spraying device, obtaining a set of sprayed particles, said spraying immediately following the contacting step, said spraying device being identical to that used for contacting, in particular a device comprising a rotating spraying element;
- spray-drying said set of sprayed particles, during which said precipitation continues until there is complete solidification of the particle, to obtain a stable pulverulent composition;
- recovery of said pulverulent composition.

8. Method according to any one of claims 4 to 7, wherein:
- said contact takes place by mixing an aqueous medium, in particular an aqueous solution, containing said acid, and an aqueous medium, in particular a solution or an aqueous suspension containing said metal or cation, and/or
- the contact of said acid with a mineral source of said metal or corresponding cation is carried out continuously, said continuous contacting in particular being carried out in a device such as static or dynamic mixers, in particular kneaders, extruders, and mixers without an internal component, such as ultrasonic mixers.

9. Method according to any one of claims 4 to 8, wherein said complex is of the following formula (Ia):
(Acid)ₙM (Ia)
where M represents said metal,
said complex being in particular a salt, more particularly a salt of formula (HMTBA)₂Ca.

10. Method according to any one of claims 4 to 9, wherein said particles consist essentially of a salt between an anion of an acid and at least one metal cation, in particular:
- said anion being 2-hydroxy-4-methyl-thiobutanoate and said cation being Ca²⁺, the source of Ca²⁺ being selected from lime, milk of lime, slaked lime, calcium hydrogen carbonate and calcium carbonate, or being Ca(OH)₂.

11. Method according to any one of claims 4 to 10, comprising an additional step of agglomeration, or in which said spray-drying tower is a multiple effect spray-drying tower, said step of reacting said acid with said mineral metal source also comprising agglomeration.
